(19) Europäisches Patentamt
European Patent Office
Office européen des brevets

(11) Veröffentlichungsnummer: **0 450 355 B1**

# (12) EUROPÄISCHE PATENTSCHRIFT

(45) Veröffentlichungstag der Patentschrift: **09.08.95**

(51) Int. Cl.$^{6}$: **C07D 333/38**, C07D 409/06, C07D 417/06, A01N 43/10

(21) Anmeldenummer: **91103875.0**

(22) Anmeldetag: **14.03.91**

## (54) Verbindungen zur Bekämpfung von Pflanzenkrankheiten.

(30) Priorität: **06.04.90 DE 4011172**

(43) Veröffentlichungstag der Anmeldung:
**09.10.91 Patentblatt 91/41**

(45) Bekanntmachung des Hinweises auf die Patenterteilung:
**09.08.95 Patentblatt 95/32**

(84) Benannte Vertragsstaaten:
**AT BE CH DE DK ES FR GB GR IT LI NL SE**

(56) Entgegenhaltungen:
**DE-A- 2 513 732**

**AGRICULTURAL AND BIOLOGICAL CHEMISTRY, Band 52, Nr. 1, 1988, Seiten 289-290; H. KAYAHARA et al.: "Evaluation of peptidic inhibitors of angiotensin converting enzyme by introducing various organic compounds"**

**CHEMICAL ABSTRACTS, Band 98, Nr. 1, 3. Januar 1983, Seite 431, Spalte 1, Zusammenfassung Nr. 4774b, Columbus, Ohio, US; A.M. EL-NAGGAR et al.: "Synthesis of some 2-thenoyl, 5-bromo-2-thenoyl and 5-nitro-2-thenoylamino acid derivatives and their antimicrobial activity"**

(73) Patentinhaber: **Degussa Aktiengesellschaft
Weissfrauenstrasse 9
D-60311 Frankturt (DE)**

(72) Erfinder: **Drauz, Karlheinz, Dr.
Zur Marienruhe 13
W-6463 Freigericht 1 (DE)**
Erfinder: **Martens, Jürgen, Prof.Dr.
Gärtnerstrasse 5
W-2900 Oldenburg (DE)**

**EP 0 450 355 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung beschreibt neue Substanzen der allgemeinen Formel I zur Bekämpfung von Pflanzenkrankheiten.

Die Erfindung betrifft ferner die Herstellung dieser Substanzen sowie die als Wirkstoff mindestens einer dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Gegenstand der Erfindung sind die Verbindungen der allgemeinen Formel I

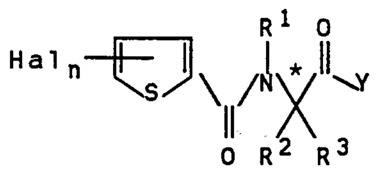

I

wobei Hal Chlor oder Brom, n die Zahl 2 oder 3,

- * bei unterschiedlichen Resten $R^2$ und $R^3$ entweder ein Enantiomeres oder ein Racemat bedeutet,
- $R^1$ für Wasserstoff, einen geradkettigen oder verzweigten Alkyl-Rest mit 1-4 C-Atomen,
- $R^2$ für Wasserstoff, einen geradkettigen Alkyl-Rest mit 1-16 C-Atomen bzw. verzweigtkettigen Alkyl-Rest mit 3-16 C-Atomen, der gegebenenfalls durch Hydroxy-, Carboxy-, $C_{1-6}$-Alkoxy- oder $C_{1-6}$-Alkoxycarbonyl, Mercapto- oder $C_{1-6}$-Alkylmercapto- oder Benzylmercapto substituiert sein kann, einen Aryl-Rest mit 6-10 C-Atomen bzw. Aralkyl-Rest mit 7-16 C-Atomen, wobei das Ringsystem gegebenenfalls mit 1-3 Fluor-, Chlor-, oder Brom-Atomen substituiert sein kann, Cycloalkyl-Rest mit 3-8 C-Atomen, Cycloalkyl-methyl-Rest mit insgesamt 4-9 C-Atomen,
- $R^3$ für Wasserstoff, einen Alkyl-Rest mit 1-4 C-Atomen, einen Alkenyl- oder Alkinyl-Rest mit 2-4 C-Atomen stehen oder gegebenenfalls $R^1$ und $R^3$ oder $R^2$ und $R^3$ zusammen eine Alkylen-Brücke mit 2-8 C-Atomen bilden können bei der gegebenenfalls eine $CH_2$-Einheiten durch $NR_1$, O oder S substituiert sein kann,
- Y OH, $OR_4$ oder

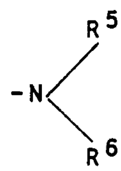

bedeutet, wobei

- $R^4$ für einen Alkyl-Rest mit 1-4 C-Atomen steht, dessen H-Atome gegebenenfalls ganz oder teilweise gegen Halogenatome ausgetauscht sind,
- $R^5$ Wasserstoff, einen Alkyl-Rest mit 1-6 C-Atomen, Hydroxy- oder einen Alkoxy-Rest mit 1-6 C-Atomen,
- $R^6$ Wasserstoff, einen Alkyl-Rest mit 1-6 C-Atomen, einen Phenyl-Rest oder einen Cycloalkyl-Rest mit 3-8 C-Atomen bedeutet oder gegebenenfalls $R^5$ und $R^6$ unter Einbeziehung des Stickstoffs-Atoms und gegebenenfalls eines weiterer Heteroatoms einen 4-8-gliedrigen Ring bilden kann.

Eine bevorzugte Ausführungsform der Erfindung betrifft Verbindungen der Formel I, die dadurch gekennzeichnet sind, daß

- $R^1$ Wasserstoff oder einen Alkyl-Rest mit 1-3 C-Atomen bedeutet,
- $R^2$ Wasserstoff, einen Alkyl-Rest mit 1-6 C-Atomen darstellt, der unsubstituiert oder durch einen Alkoxycarbonyl-Rest mit 1-6 C-Atomen substituiert sein kann, ein Phenyl-, Aralkyl mit 7 oder 8 C-Atomen oder Cycloalkyl-Rest mit 3-7 C-Atomen ist,
- $R^3$ Wasserstoff, oder Alkyl-Rest mit 1-4 C-Atomen ist oder $R^2$ und $R^3$ zusammen eine Alkylenbrücke mit 2 C-Atomen bilden und
- Y OH, $OR_4$ oder $NR_5R_6$ ist, worin

$R^4$ einen Alkyl-Rest mit 1-4 C-Atomen bedeutet, dessen Wasserstoff ganz oder teilweise gegen Fluor ausgetauscht sein kann, und

$R^5$ und $R^6$ unabhängig voneinander Wasserstoff oder Alkyl mit 1-4 C-Atomen oder Alkoxy mit 1-4 C-Atomen bedeutet oder worin $R^5$ und $R^6$ zusammen mit dem N-Atom einen 5-6-gliedrigen Ring bilden, der kein oder ein weiteres N oder O enthält.

Eine weitere bevorzugte Ausführungsform der Erfindung betrifft Verbindungen der Formel I, die dadurch gekennzeichnet sind, daß Hal Brom, n die Zahl 2 und $R^1$ Wasserstoff bedeuten.

Eine weitere bevorzugte Ausführungsform der Erfindung betrifft Verbindungen der Formel I, die dadurch gekennzeichnet sind, daß

$R^2$ Wasserstoff oder einen Alkyl-Rest mit 1-4 C-Atomen bedeutet, der unsubstituiert oder durch einen Alkoxycarbonyl-Rest mit 1-2 C-Atomen substituiert sein kann,

$R^3$ Wasserstoff-, Methyl-, oder Ethyl ist, und

Y OH, $OR^4$- oder $NR_5R_6$ bedeutet, worin $R^4$ einen Alkyl-Rest mit 1-4 C-Atomen und $R^5$ und $R^6$ unabhängig voneinander Wasserstoff oder einen Alkyl-Rest mit 1-4 C-Atomen bedeuten.

Eine weitere bevorzugte Ausführungsform der Erfindung betrifft Verbindungen der Formel I, die dadurch gekennzeichnet sind, daß

$Hal_n$ 2 Bromatome in Position 2' und 3' des Thiophenrings bedeuten.

Ein weiterer Gegenstand der Erfindung ist die Herstellung von Verbindungen der allgemeinen Formel I, die dadurch gekennzeichnet ist, daß man ein Säurehalogenid der allgemeinen Formel II

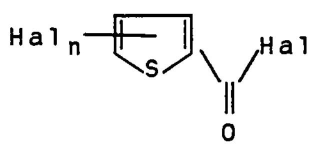

II

Hal = Cl, Br

n = 2 oder 3

mit einer Aminokomponente der allgemeinen Formel III

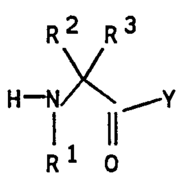

III

in der $R^1$, $R^2$, $R^3$ und Y die angegebenen Bedeutungen haben, in Gegenwart einer Base umsetzt.

Weiterhin ist ein Gegenstand der Erfindung, daß Mittel zur Bekämpfung von pflanzenpathogenen Keimen und zur Verhinderung von Krankheitsbefall an Pflanzen, als aktive Komponente mindestens eine Verbindung der Formel I und in der Formulierungstechnik übliche Hilfmittel enthalten.

Außerdem sind ein Gegenstand der Erfindung Verfahren zur Herstellung von Mitteln zur Bekämpfung von pflanzenpathogenen Keimen und zur Verhinderung von Krankheitsbefall an Pflanzen, die als aktive Komponente mindestens eine Verbindung gemäß der Formel I und in der Formulierungstechnik übliche Hilfmittel.

Weiterhin wurde gefunden, daß Verbindungen der Formel I zur Bekämpfung von pflanzenpathogenen Keimen und zur Verhüntung von Krankheitsbefall an Pflanzen verwendet werden können.

Schließlich wurde ein Verfahren gefunden, bei dem Pflanzen vor Krankheitsbefall geschützt wurden durch die Behandlung der Pflanze, ihrer Pflanzenteile oder des Orts ihres Wachstums mit einer wirksamen Menge einer Verbindung der Formel I.

Die Verbindungen der allgemeinen Formel II lassen sich nach bekannten Methoden, ausgehend von den Carbonsäuren IV herstellen, die ihrerseits ebenfalls nach literaturbekannten Methoden gewonnen werden[1-11].

1. D. Pillon et. al. Chimie Therapeutique 1, 1970,32
2. DOS 1 813 195
3. Französische Patentanmeldung 1 563 735
4. US-Pat. 3 887 354

5. US-Pat. 3 536 473
6. US-Pat. 3 303 201
7. J.A. 7 014 560-R
8. EP 216 279
9. Ber. Dtsch. Chem. Ges. 18, 2308 (1885)
10. Liebigs. Ann. Chem. 532, 250 (1937)
11. ibid 511/512 S. 136, 1934.
12. J. Indian Chem. Soc. 59, 783 (1982)

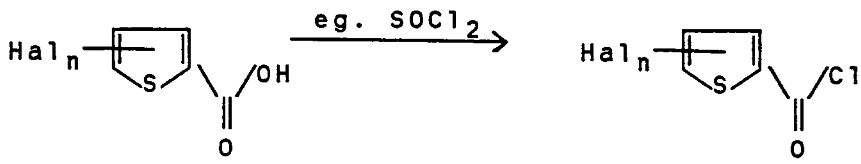


IV II

Speziell das 2.3-Dibromthiophen-5-carbonsäurebromid IIa läßt sich direkt und in guter Ausbeute gewinnen durch Umsetzung der Thiophen-2-carbonsäure mit Thionylchlorid, Reaktion des erhaltenen Thiophen-2-carbonsäurechlorids mit elementarem Brom bei 90 - 100 °C, Isolierung des Säurebromids und anschließende fraktionierte Destillation im Hochvakuum.

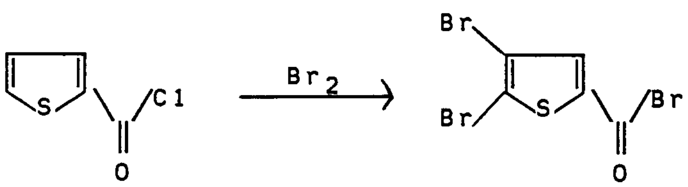


IIa

Die Verbindungen der allgemeinen Formel III werden aus den entsprechenden optisch aktiven oder racemischen Aminosäuren hergestellt.

Die Aminosäureester III (Y = $OR^4$) werden ebenfalls nach literaturbekannten Verfahren hergestellt (Houben-Weyl, Band 15/1, Seite 315 - 338; Georg Thieme Verlag, Stuttgart 1974; parallele Anmeldung (P 40 11 171.7), die Herstellung der Aminosäureamide III (Y = $NR^5R^6$) gelingt durch a) Umsetzung von N-geschützten $\alpha$-Aminosäuremethylestern mit Ammoniak bzw. Aminen, insbesondere Alkylaminen oder durch Umsetzung von Aktivestern von N-geschützten $\alpha$-Aminosäuren, wie z.B. p-Nitrophenylestern bzw. gemischten Anhydriden, herstellbar aus N-geschützten $\alpha$-Aminosäuresalzen der Alkalimetalle und Säurechloriden bzw. Chlorameisensäureestern mit den entsprechenden Aminokomponenten und anschließender Abspaltung der N-Schutzgruppen (siehe Houben-Weyl, Band 15/1, Seite 453 - 457, Georg Thieme Verlag, Stuttgart 1974) oder b) durch Umsetzung von N-Carboxyanhydriden, deren Herstellung z.B. in H.R. Kricheldorf, $\alpha$-Aminoacid-N-Carboxy-Anhydrides and Related Heterocycles, Springer-Verlag, Berlin, Heidelberg, 1987, Seite 3- 51 beschrieben wird, mit weniger reaktionsfähigen Aminen. (ibid, Seite 59 - 71).

Die Umsetzung der Säurehalogenide der allg. Formel II mit den Aminen der allg. Formel III erfolgt vorzugsweise in Wasser oder organischen Lösungsmitteln wie z.B. Carbonsäureestern, halogenierten Kohlenwasserstoffen, Ethern bzw. Mischungen davon; als Basen finden vorzugsweise NaOH, KOH, Carbonate und Hydrogencarbonate sowie tert. Amine und Pyridin Einsatz. Diese Basen können gleichzeitig als Lösungsmittel dienen ( Beispiel 35). Die Temperatur der Umsetzung ist nicht weiter kritisch, je nach Reaktivität der Komponente ist ein Temperaturbereich zwischen -10°C und dem Siedepunkt des jeweiligen Lösungsmittels bzw. -gemisch zu wählen. Zweckmäßigerweise erfolgen die Umsetzungen bei 5°C - 25°C.
Die Reaktionskomponenten und die Base werden vorzugsweise in stöchiometrischen Mengen umgesetzt. Dabei wird so verfahren, daß man das Säurehalogenid der allgemeinen Formel II im jeweiligen Lösungsmittel zu einer Suspension bzw. Lösung der Aminoverbindungen der allg. Formel III und der Base im Lösungsmittel zudosiert. Nach erfolgter Umsetzung wird, sofern man in rein organischen Systemen arbeitet, vom Aminhydrochlorid abfiltriert, das Lösungsmittel abdestilliert, der Rückstand mit Wasser gewaschen und ggf. mit einem weiteren Lösungsmittel zur Kristallisation gebracht. Bei Arbeiten in wasser oder wäßrigen

Gemischen wird entweder das feste Produkt filtriert und mit Wasser nachgewaschen, bzw. die erhaltenen Öle mit einem mit Wasser nicht mischbaren Lösungsmittel extrahiert, dieses Lösungsmittel mit wasser neutral gewaschen , getrocknet und abdestilliert. Es verbleiben ebenfalls feste Rückstände, die direkt oder nach Waschen bzw. Umkristallisieren ausreichende Reinheit aufweisen. Die erhaltenen Produkte werden dann bei Temperaturen zwischen 20 - 50 °C bis zur Gewichtskonstanz getrocknet.

Es wurde nun überraschenderweise gefunden, daß die erfindungsgemäßen Verbindungen der Formel I durch ihre Anwendung den Befall von Pflanzen durch schädliche Mikroorganismen verhindern und damit den befallsbedingten Schädigungen der Pflanzen vorbeugen.

Für die erfindungsgemäßen Wirkstoffe ist charakteristisch, daß der Schutz der Pflanzen sowohl durch direkte Einwirkung auf die pflanzenschädigenden Mikroorganismen mittels Blattapplikationen (direkte Wirkung) oder Bodenapplikationen (systemische Wirkung) eintreten kann. Aufgrund der sehr guten Wirkungsweise der erfindungsgemäßen Verbindungen der Formel I, kann ein breit gefächerter Schutz der Pflanzen gegen Krankheiten erzielt werden. Die Anwendung der erfindungsgemäßen Wirkstoffe ist ist deshalb besonders für praktische Bedingungen geeignet. Darüber hinaus bewirkt die den Verbindungen der Formel I zueigene systemische Aktivität, daß sich der Schutzeffekt auch auf zuwachsende Pflanzenteile der behandelten Pflanzen erstreckt.

Die allgemein pflanzenschützende Aktivität der erfindungsgemäßen Wirkstoffe ist z.B. gegen die folgenden Klassen phytopathogener Fungi wirksam: Fungi imperfecti (z.B. Botrytis, Helminthosporium, Fusarium, Septoria, Cercospora und Alternaria); Basidiomyceten (z.B. die Gattungen Hemileia, Rhizoctonia, Puccinia); Ascomyceten (z.B. Venturia, Podosphaera, Erysiphe, Monilia, Uncinula).

Darüberhinaus können die Wirkstoffe besonders vorteilhaft gegen folgende Schadorganismen eingesetzt werden: Pilze, wie z.B. Oomyceten (z.B. Plasmopara viticola, Phytophthora infestans, Peronospora tabacina, Pseudoperonospora), Fungi imperfecti (z.B. Colletotrichum lagenarium, Pyricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemäßen Verbindungen können zum Schutz von Pflanzen aus unterschiedlichen Nutzkulturen eingesetzt werden.

Für den Einsatz der erfindungsgemäßen Verbindungen sind beispielsweise folgende Pflanzenarten geeignet: Getreide (Weizen, Gerste, Roggen, Hafer, Reis, Sorghum und Verwandte); Rüben (Zucker- und und Futterrüben); Kern-, Stein- und Beerenobst (Äpfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Ölkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika);

Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemäßen Verfahrens sind folgende Pflanzen anzusehen: Gurke, Tabak, Reben, Reis, Birne, Pfeffer, Kartoffeln, Tomate und Apfel.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentrationen, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ und $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -Ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$ - $C_{22}$), wie die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze geeignet.

Häufiger werden jedoch sogenannte synthetische Tenside verwendet, insbesondere Fettsulfonate, Fettsulfate, sulfonierte Benzimidazolderivate oder Alkylarylsulfate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Ethylenoxid-Addukten. Die sulfonierten Benzimidazolderivate enthalten vorzugsweise 2 Sulfonsäuregruppen und einen Fettsäurerest mit 8 bis 22 C-Atomen. Alkylarylsulfonate sind z.B. die Na-, Ca-oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate z.B. Salze des Phosphorsäureesters eines p-Nonylphenol-(4-14)-ethylen-oxid-Adduktes und Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylenphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxid-Addukte an Polypropylenglykol, Ethylendiaminopropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglycolether, Polypropylen-Polyethylenoxid-Addukte, Tributylphenoxypolyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Rigewood, New Jersey, 1979; Dr. Helmut Stache "Tensid Taschenbuch", Carl Hanser Verlag, München/Wien 1981.

Die pestiziden Zubereitungen enthalten in der Regel 0,1 bis 99 Gew.%, insbesondere 0,1 bis 95 Gew.%, Wirkstoff der Formel I, 1 bis 99,9 Gew.% eines festen oder flüssigen Zusatzstoffes und 0 bis 25 Gew.%, insbesondere 0,1 bis 25 Gew.%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatore, Bindemittel, Haftmittel sowie Dünger oder andere Wirkstoffe zur Erzielung spezieller Effekte enthalten.

FORMULIERUNGSBEISPIELE

Formulierungsbeispiele für flüssige Wirkstoffe der Formel I % = Gewichtsprozent)

Emulsions-Konzentrate

| | a) | b) | c) |
|---|---|---|---|
| Wirkstoff aus den Tabellen | 25 % | 40 % | 50 % |
| Ca-Dodecylbenzolsulfonat | 5 % | 8 % | 6 % |
| Rizinusöl-polyethylenglykolether (36 Mol Ethylenoxid) | 5 % | -- | -- |
| Tributylphenoyl-polyethylenglykolether (30 Mol Ethylenoxid) | -- % | 12 % | 4 % |
| Cyclohexanon | -- | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

Lösungen

| | a) | b) | c) | d) |
|---|---|---|---|---|
| Wirkstoff aus den Tabellen | 80 % | 10 % | 5 % | 95 % |
| Ethylenglykolmonomethylether | 20 % | -- | -- | -- |
| Polyethylenglykol MG 400 | -- | 70 % | -- | -- |
| N-Methyl-2-pyrrolidon | -- | 20 % | -- | -- |
| Epoxidiertes Kokosnußöl | -- | -- | 1 % | 5 % |
| Benzin (Siedegrenzen 160-190°C) | -- | -- | 94 % | -- |

(MG = Molekulargewicht)

Die Lösungen sind zur Anwendung in Form kleinster Tropfen geeignet.

Granulate

| | a) | b) |
|---|---|---|
| Wirkstoff aus den Tabellen | 5 % | 10 % |
| Kaolin | 94 % | -- |
| Hochdisperse Kieselsäure | 1 % | -- |
| Attapulgit | -- | 90 % |

Der Wirkstoff wird in Methylenchlorid gelöst, auf den Träger aufgesprüht und das Lösungsmittel anschließend in Vakuum abgedampft.

Stäubemittel

| | a) | b) |
|---|---|---|
| Wirkstoff aus den Tabellen | 2 % | 5 % |
| Hochdisperse Kieselsäure | 1 % | 5 % |
| Talkum | 97 % | -- |
| Kaolin | -- | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

Spritzpulver

| | a) | b) | c) |
|---|---|---|---|
| Wirkstoff aus den Tabellen | 25 % | 40 % | 50 % |
| Na-Ligninsulfonat | 5 % | 8 % | 6 % |
| Na-Laurylsulfat | 5 % | 27 % | 24 % |
| Na-Diisobutylnaphthalinsulfonat | -- | -- | -- |
| Octylphenolpolyethylenglycolether (7-8 Mol Ethylenoxid) | -- | -- | -- |
| Hochdispersive Kieselsäure | 65 % | 25 % | 20 % |
| Kaolin | -- | -- | -- |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

Emulsions-Konzentrate

| | |
|---|---|
| Wirkstoff aus den Tabellen | 10 % |
| Octylphenolpolyethylenglycolether (4-5 Mol Ethylenoxid) | 3 % |
| Ca-Dodecylbenzolsulfonat | 3 % |
| Rizinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

Stäubemittel

| | a) | b) |
|---|---|---|
| Wirkstoff aus den Tabellen | 5 % | 8 % |
| Talkum | 95 % | -- |
| Kaolin | -- | 92 % |

den Trägerstoffen vermischt und auf einer geeigneten Mühle vermahlen wird.

Extruder-Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend in Luftstrom getrocknet.

Umhüllungs-Granulat

| | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

Suspension-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37 %ige wäßrige Formaldehyd-Lösung | 0,2 % |
| Silikonöl in Form einer 75 %igen wäßrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

Beispiel 1

670 g Thiophen-2-carbonsäure werden mit 1243 g (760 ml) frisch destilliertem Thionylchlorid unter Rühren vermischt und langsam erwärmt. Nach 2.5 h bei 30 °C entsteht eine klare Lösung. Danach wird weitere 2 h unter Rückfluß gerührt. Danach wird das überschüssige Thionylchlorid abdestilliert und bei 83 °C/20 mbar gehen 734 g (95.7 % d.Th.) Thiophen-2-carbonsäurechlorid über.

605 g Thiophen-2-carbonsäurechlorid werden auf 110 °Cerwärmt und unter Rühren innerhalb von 90 min 2077 g (668 ml) Brom zugetropft, worauf HBr-Gas entweicht. Anschließend wird noch weitere 148 h bei 100 °C gerührt. Nach Reaktionsende wird durch Anlegen eines leichten Vakuums von restlichen HBr und Bromspuren befreit und anschließend fraktioniert destilliert. Bei 94 °C und 0.2 mbar erhält man 1236 g einer Fraktion (85.8 % d.Th.) 2.3-Dibromthiophen-5-carbonsäurebromid. Schmelzpunkt 38 - 40 °C, die elementar-

analytischen Werte entsprechen den kalkulierten Werten.

$^1$H-NMR ($CDCl_3$): 7.75 ppm (s, 1H)

8.0 g 2.3-Dibromthiophen-5-carbonsäurebromid, gelöst in 50 ml THF, werden zu einer Suspension von 2.88 g Glycinmethylester•HCl und 4.62 g Triethylamin in 100 ml THF bei 20 - 25 °C innerhalb von 3 h zugetropft. Die entstandene dicke Suspension wird noch weitere 2 h bei der gleichen Temperatur nachgerührt, filtriert und das Filtrat in Wasserstrahlvakuum eingedampft.

Der so erhaltene Rückstand wird 2 x mit je 50 ml $H_2O$ ausgerührt und nach Abfiltrieren mit 100 ml Petrolether gewaschen und anschließend bei 40 °C/30 mbar bis zur Gewichtskonstanz getrocknet.

Man erhält 5.3 g (71.1 % d.Th.) H-(2'.3'-Dibrom-5'-thenoyl)-glycinmethylester mit einem Schmelzpunkt von 97 - 99 °C.

## Beispiel 2

Zu einer Lösung von 6.6 g Glycinisopropylester•HCl und 8.7 g Triethylamin in 10 ml THF werden innerhalb von 3 h bei 25 °C 15 g 2.3-Dibromthiophen-5-carbonsäurebromid, gelöst in 50 ml THF, zugetropft. Man rührt 2 h bei 25 °C nach, saugt vom ausgefallenen Feststoff ab, dampft das Filtrat ein und entfernt restliches Lösungsmittel im Hochvakuum, wobei Kristallisation eintritt. Die Kristalle werden mit n-Pentan gewaschen, abfiltriert und bei 50 °C/30 mbar bis zur Gewichtskonstanz getrocknet.

Man erhält 15.1 g (88.1 % d.Th.) N-(2'.3'-Dibrom-5'-thenoyl)-glycinisopropylester mit einem Schmelzpunkt von 100 - 102 °C.

## Beispiel 3

Eine Suspension 1.87 g D.L-Alaninisopropylester•HCl und 2.25 g Triethylamin in 100 ml THF werden mit einer Lösung von 3.89 g 2.3-Dibromthiophen-5-carbonsäurebromid in 50 ml THF gemäß Beispiel 1 umgesetzt.

Nach Aufarbeitung erhält man 3.26 g (73.3 % d.Th.) N-(2'.3'-Dibrom-5'-thenoyl)-D.L-alaninisopropylester mit einem Schmelzpunkt von 117 - 118 °C.

## Beispiel 4

Eine Suspension von 2.20 g α-Aminoisobuttersäuremethylester•HCl und 2.89 g Triethylamin in 100 ml Diisopropylether werden mit einer Lösung von 5.0 g 2.3-Dibromthiophen-5-carbonsäurebromid in 50 ml Diisopropylether umgesetzt.

Nach Aufarbeitung erhält man 4.1 g (74.1 % d.Th.) N-(2'.3'-Dibrom-5'-thenoyl)-2-aminoisobuttersäuremethylester mit einem Schmelzpunkt von 137 - 138 °C.

## Beispiel 5

Eine Suspension von 3.1 g 1-Aminocyclohexancarbonsäuremethylester•HCl und 3.99 g Triethylamin in 100 ml Methyl-tert.-butylether (MTBE) werden mit einer Lösung von 6.0 g 2.3-Dibromthiophen-5-carbonsäurechlorid gemäß Beispiel 1 umgesetzt.

Nach Aufarbeitung erhält man 6.0 g (72 % d.Th.) (2'.3'-Dibrom-5'-thenoyl)-1-aminocyclohexancarbonsäur e methylester mit einem Schmelzpunkt von 149 -150 °C.

## Beispiel 6

Die Lösung von 7.2 g L-Valinmethylester•HCl mit 8.7 g Triethylamin in 100 ml THF wird gemäß Beispiel 2 mit einer Lösung von 15 g 2.3-Dibromthiophen-5-carbonsäurebromid in 50 ml THF umgesetzt.

Nach Aufarbeitung erhält man 15.1 g (88.1 % d.Th.) N-(2'.3'-Dibrom-5'-thenoyl)-L-valinmethylester mit einem Schmelzpunkt von 100 - 102 °C.

## Beispiel 7

Gemäß Beispiel 6 werden 7.2 g D-Valinmethylester•HCl, 8.7 g Triethylamin und 15 g 2.3-Dibromthiophen-5-carbonsäurebromid in insgesamt 150 ml THF umgesetzt.

Nach Aufarbeitung erhält man 14.9 g (86.8 % d.Th.) N-(2'.3'-Dibrom-5'-thenoyl)-D-valinmethylester mit einem Schmelzpunkt von 99 - 101 °C.

Beispiel 8

Gemäß Beispiel 1 wird die Suspension von 2.36 g L-Valin-2'.2'.2'-trifluorethylester•HCl und 2.02 g Triethylamin in 100 ml THF mit einer Lösung von 3.04 g 2.3-Dibromthiophen-5-carbonsäurechlorid in 50 ml THF umgesetzt.

Nach Aufarbeitung erhält man 3.42 g (73.2 % d.Th.) N-(2'.3'-Dibrom-5'-thenoyl)-L-valin-2''.2''.2''-trifluor ethylester mit einem Schmelzpunkt von 52 - 53 °C.

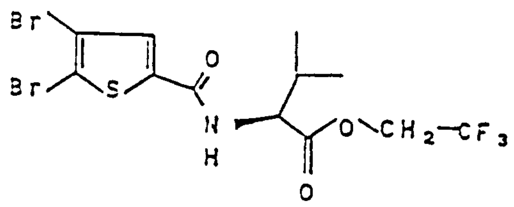

Beispiel 9

Gemäß Beispiel 1 wird die Suspension von 4.13 g L-Valin-n-butylester•HCl und 4.0 g Triethylamin in 100 ml THF mit der Lösung von 6.0 g 2.3-Dibromthiophen-5-carbonsäurechlorid in 50 ml THF umgesetzt.

Nach Aufarbeitung erhält man 7.12 g (81.9 % d. Th.) N-(2'.3'-Dibrom-5'-thenoyl)-L-valin-n-butylester als viskoses öl.

Beispiel 10-30 werden analog Beispiel 1 umgesetzt (s. Tab. 1).

Tabelle 1:

| Beispiel | AS-Ester·HCl o. Amid·HCl [g] | 2.3-Dibromthiophen-5-carbonsäurehalogenid [g] | Base [g] | Lsgm. [ml] | Produkt Ausbeute [g]/Fp |
|---|---|---|---|---|---|
| 10 | 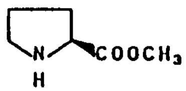 1.5 | -bromid, 4.0 | $NEt_3$, 2.31 | THF, 150 | 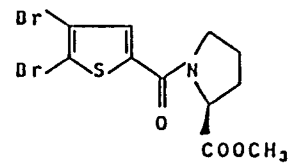 4.0 / 107-109°C |
| 11 | 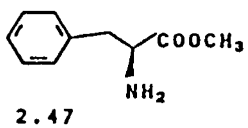 2.47 | -bromid 4.0 | $NEt_3$, 2.31 | THF, 150 | 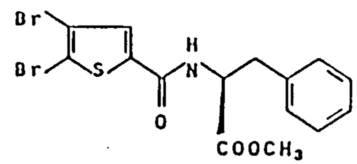 3.1 / viskoses Öl |
| 12 | 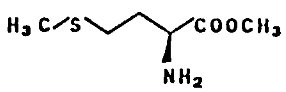 9.57 | -bromid 15.0 | $NEt_3$, 0.7 | THF, 150 | 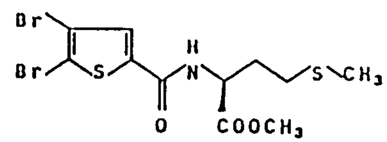 14.5 / viskoses Öl |
| 13 | 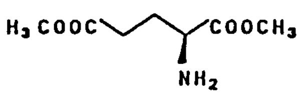 2.43 | -bromid 4.0 | $NEt_3$, 2.31 | THF, 150 | 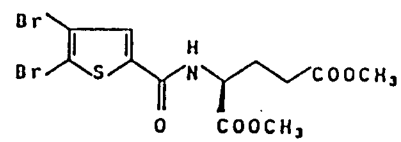 3.0 / viskoses Öl |

| Beispiel | AS-Ester·HCl o. Amid·HCl [g] | 2.3-Dibromthiophen-5-carbonsäurehalogenid [g] | Base [g] | Lsgm. [ml] | Produkt Ausbeute [g]/Fp |
|---|---|---|---|---|---|
| 14 | 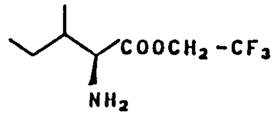 3.29 | -chlorid 3.97 | $NEt_3$, 2.63 | THF, 150 | 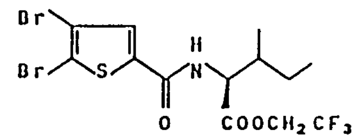 4.88 / 84-5°C |
| 15 | 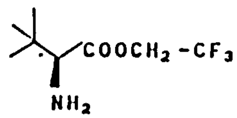 3.29 | -chlorid 3.97 | $NEt_3$, 2.63 | THF, 150 | 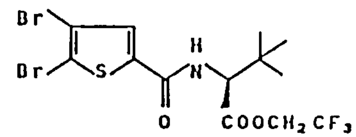 5.53 / 131-3°C |
| 16 | 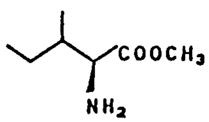 11.7 | -chlorid, 22.5 | $NEt_3$, 13.05 | THF, 150 | 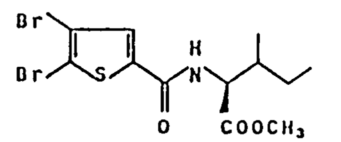 22.0 / 57-9°C |
| 17 | 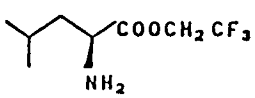 3.29 | -chlorid, 3.97 | $NEt_3$, 2.63 | THF, 150 | 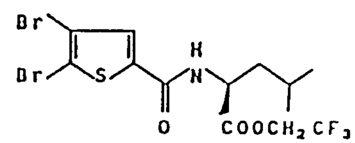 4.61 / 79-81°C |

| Beispiel | AS-Ester·HCl o. Amid·HCl [g] | 2.3-Dibromthiophen-5-carbonsäurehalogenid [g] | Base [g] | Lsgm. [ml] | Produkt Ausbeute [g]/Fp |
|---|---|---|---|---|---|
| 18 | 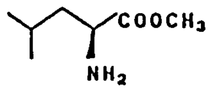 7.01 | -bromid, 15.0 | $NEt_3$, 0.7 | THF, 150 | 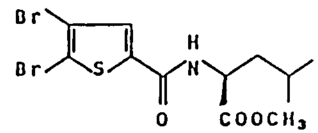 16.1 / 100-02°C |
| 19 | 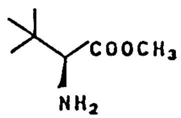 7.01 | -bromid 15.0 | $NEt_3$, 0.7 | THF, 150 | 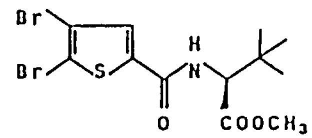 14.7 / 125-128°C |
| 20 | 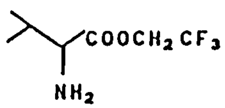 2.36 | -chlorid 3.04 | $NEt_3$, 2.02 | THF, 150 | 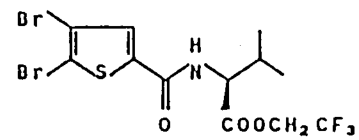 3.42 / 52-3°C |
| 21 | 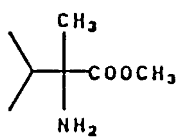 3.12 | -chlorid 5.23 | $NEt_3$, 3.40 | THF, 100 | 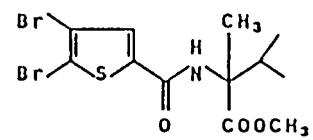 5.8 / 107-109°C |

| Beispiel | AS-Ester·HCl o. Amid·HCl [g] | 2.3-Dibromthiophen-5-carbonsäurehalogenid [g] | Base [g] | Lsgm. [ml] | Produkt Ausbeute [g]/Fp |
|---|---|---|---|---|---|
| 22 | 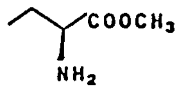 2.68 | -chlorid, 5.23 | $NaHCO_3$ 2.00 | $H_2O$, 100 THF, 50 | 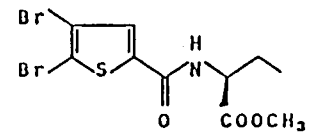 5.96 / 60-70°C |
| 23 | 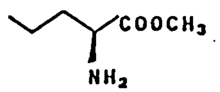 2.00 | -chlorid, 5.23 | $NEt_3$ 3,40 | THF, 150 | 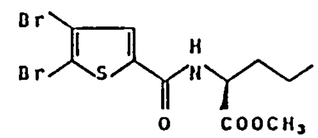 6.09 / 79-81°C |
| 24 | 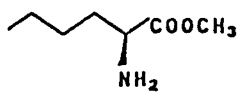 3.10 | -chlorid, 5,23 | $NEt_3$ 3.40 | THF, 150 | 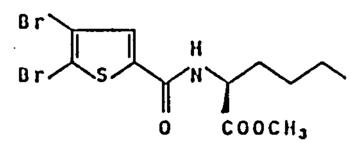 6.15 / 59-61°C |
| 25 | 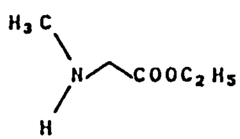 2.64 | -chlorid, 5.23 | $NEt_3$ 3.40 | THF, 150 | 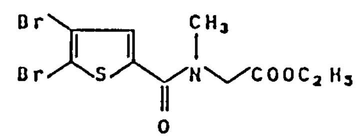 5.63 / 80-82°C |

| Beispiel | AS-Ester·HCl o. Amid·HCl [g] | 2.3-Dibromthiophen-5-carbonsäurehalogenid [g] | Base [g] | Lsgm. [ml] | Produkt Ausbeute [g]/Fp |
|---|---|---|---|---|---|
| 26 | 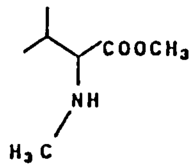 | -chlorid, 5.23 | $NEt_3$ 3.40 | THF, 150 | 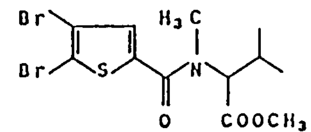 |
| 27 | 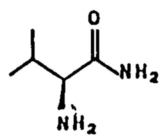 50 | -chlorid, 99.7 | $NaHCO_3$ 55 | $H_2O$, 1500 THF, 700 | 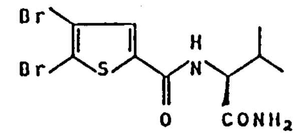 120 / 238-240°C |
| 28 | 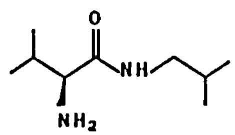 3.98 | -chlorid, 5.23 | $NaHCO_3$ 2.00 | $H_2O$, 100 THF, 50 | 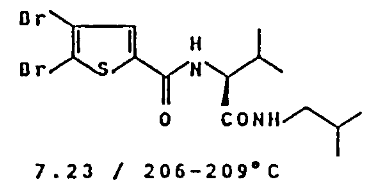 7.23 / 206-209°C |

| Beispiel | AS-Ester·HCl o. Amid·HCl [g] | 2.3-Dibromthiophen 5-carbonsäurehalogenid [g] | Base [g] | Lsgm. [ml] | Produkt Ausbeute [g]/Fp |
|---|---|---|---|---|---|
| 29 | 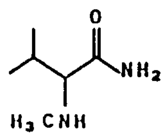 2.07 | -chlorid, 5.23 | $NaHCO_3$ 2.08 | $H_2O$, 100 THF, 50 | 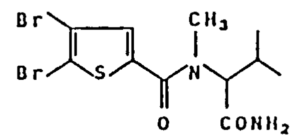 5.79 / 150-152°C |
| 30 | 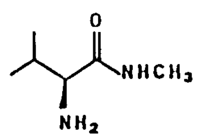 2.0 (als Acetat) | -chlorid, 3.2 | $NaHCO_3$ 1.77 | $H_2O$, 100 THF, 35 | 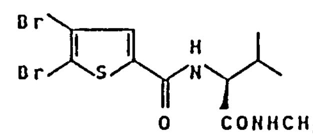 3.98 / 230-231°C |

Beispiel 31

4.0 g 2.3-Dibromthiophen-5-carbonsäurebromid, gelöst in 50 ml THF, werden zu einer Suspension von 2.89 g L-Valinanilidacetat und 2.31 g Triethylamin in 100 ml THF bei 25 °C innerhalb von 3 h zugetropft. Die so erhaltene dicke Suspension wird noch weitere 2 h bei dieser Temperatur gerührt, filtriert und das

Filtrat bei 40 mbar eingedampft.
Der so erhaltene Rückstand wird 2 x mit 30 ml $H_2O$ ausgewaschen und nach Filtration mit 50 ml Petrolether (50 - 90 °C) und anschließend bei 50 °C bis zur Gewichtskonstanz getrocknet.

Man erhält 4.0 g (75.8 % d.Th.) $N_\alpha$-(2'.3'-Dibrom-5'-thenoyl)-L-valinanilid mit einem Schmelzpunkt von 195 - 198 °C.

Beispiel 32

4.0 g 2.3-Dibromthiophen-5-carbonsäurebromid werden gemäß Beispiel 31 mit 2.94 g L-Leucinanilidacetat umgesetzt.

Nach Aufarbeitung erhält man 2.5 g (50 % d.Th.) $N_\alpha$-(2'.3'-Dibrom-5'-thenoyl)-L-leucinanilid mit einem Schmelzpunkt von 210 - 212 °C.

Beispiel 33

Gemäß Beispiel 31 wird die Suspension von 1.59 g L-Valinamid•HCl und 2.31 g Triethylamin in 100 ml THF mit der Lösung von 4.0 g 2.3-Dibromthiophen-5-carbonsäurechlorid in 25 ml THF umgesetzt.

Nach Aufarbeitung erhält man 3.27 g (74.2 % d.Th.) $N_\alpha$-(2'.3'-Dibrom-5'-thenoyl)-L-valinamid mit einem Schmelzpunkt von 235 - 40 °C.

Beispiel 34

5.23 g 2.3-Dibromthiophen-5-carbonsäurechlorid in 50 ml THF werden zu einer Suspension von 3.81 g L-Hexahydrophenyl-alaninmethylester•HCl und 2.88 g $NaHCO_3$ in 100 ml Wasser bei 10 °C innerhalb von einer Stunde zudosiert.

Nach weiteren 2 h Nachrühren wird das abgeschiedene Öl in 100 ml Essigester aufgenommen, über $Na_2SO_4$ getrocknet und das Lösungsmittel abdestilliert. Der viskose Rückstand wird bei 0.1 mbar und 50 °C von Lösungsmittelresten befreit. Man erhält 6.3 g (80.1 % d.Th.) N-(2'.3'-Dibrom-5'-thenoyl)-L-hexahydrophenyl-alaninmethylester als viskoses Öl.

Beispiel 35

Zu einer Lösung von 6.71 g L-Valinmethylester•HCl in 50 ml Pyridin wird eine Lösung von 15.3 g 2.3.4-Tribromthiophencarbonsäurechlorid in 50 ml THF innerhalb 30 min bei 20 °C zudosiert. Nach zweistündigem Nachrühren bei derselben Temperatur filtriert man die ausgefallenen Salze ab und wäscht den Rückstand mit 3 x 20 ml $H_2O$ neutral. Man nimmt in 200 ml heißem Aceton auf, versetzt mit 5 g Aktivkohle und filtriert über ein Druckfilter ab. Beim Eindampfen der geklärten Lösung erhält man 10.8 g (56.4 % d.Th.) N-(2'.3'.4'-Tribromthenoyl)-L-valinmethylester mit einem Schmelzpunkt von 110 - 112 °C.

Beispiel 36

Gemäß Beispiel 35 werden 4.36 g D.L-Valinmethylester•HCl in 30 ml Pyridin mit einer Lösung von 10 g 2.3.4-Tribromthiophencarbonsäurechlorid in 30 ml THF umgesetzt.

Nach Umkristallisation aus einem 1:1-Gemisch aus Petrolether/$Et_2O$ erhält man 1.7 g (13.7 % d.Th.) N-(2'.3'.4'-Tribromthenoyl)-D.L-valinmethylester mit einem Schmelzpunkt von 85 - 88 °C.

Beispiel 37

Gemäß Beispiel 35 werden 5.66 g L-Leucinmethylester•HCl in 30 ml Pyridin mit 15 g 2.3.4-Tribromthiophencarbonsäurechlorid in 40 ml THF umgesetzt.

Nach Aufarbeitung erhält man 4.8 g (25 % d.Th.) N-(2'.3'.4'-Tri-bromthenoyl)-L-leucinmethylester mit einem Schmelzpunkt von 70 - 3 °C.

Beispiel 38

Gemäß Beispiel 35 werden 7.26 g L-Valinethylester•HCl in 50 ml Pyridin mit 15.3 g 2.3.4-Tribromthiophencarbonsäurechlorid in 50 ml THF umgesetzt.

Nach Aufarbeitung erhält man 11.8 g (60 % d.Th.) N-(2'.3'.4'-Tribromthenoyl)-L-valinethylester mit einem Schmelzpunkt von 102 - 05 °C.

Beispiel 39

Gemäß Beispiel 35 werden 8.3 g 1-Aminocycloheptancarbonsäuremethylester•HCl in 50 ml Pyridin mit 15.3 g 2.3.4-Tribromthiophencarbonsäurechlorid in 50 ml THF umgesetzt.

Nach Aufarbeitung erhält man 8.1 g (39.0 % d.Th.) N-(2'.3'.4'-Tribromthenoyl)-1-aminocycloheptancarbonsäuremethylester mit einem Schmelzpunkt von 165 - 68 °C.

Beispiel 40

In eine Lösung von 2.27 g KOH und 2.25 g N-Methyl-D.L-valin in 100 ml $H_2O$ werden bei 20 °C. innerhalb von 2 h 2.23 g 2.3-Dibromthiophen-5-carbonsäurechlorid in 40 ml Dioxan zugetropft. Nach einstündiger Nachreaktion wird mit HCl auf pH 2 angesäuert, die organische Phase abgetrennt und eingedampft. Der Rückstand wird mit 30 ml Diisopropylether kristallisiert. Man erhält 5.14 g (74.9 % d.Th.) N-(2'.3'-Dibrom-5'-thenoyl)-N-methyl-D.L-valin mit einem Schmelzpunkt 160 - 163 °C.

Beispiel 41

5.0 g 2.3.4-Tribromthiophencarbonsäurechlorid, gelöst in 50 ml THF, werden zu einer Suspension von 3.29 g L-tert.-Leucin-2'.2'.2'-trifluorethylester•HCl und 2.63 g Triethylamin in 100 ml THF innerhalb von 3 h bei 20 °C zudosiert. Die so erhaltene dicke Suspension wird noch weitere 2 h bei dieser Temperatur gerührt, filtriert und das Filtrat bei 40 mbar eingedampft. Der so erhaltene Rückstand wird 2 x mit 30 ml $H_2O$ und anschließend noch mit 50 ml Petrolether/$Et_2O$ (2:1) gewaschen und bis zur Gewichtskonstanz getrocknet.

Man erhält 5.26 g (72 % d.Th.) N-(2'.3'.4'-Tribromthenoyl)-L-tert.-leucin-2''.2''.2''-trifluorethylester mit einem Schmelzpunkt von 93 - 5 °C.

Beispiele 42-70 werden analog Beispiel 41 umgesetzt (s. Tab. 2).

Tabelle 2:

| Beispiel | AS-Ester·HCl o. Amid·HCl | 2.3.4.-Tribromthiophen-carbonsäurechlorid [g] | $NEt_3$ [g] | Lsgm. [ml] | Produkt/Fp |
|---|---|---|---|---|---|
| 42 | 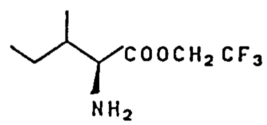 3.29 | 5.0 | $NEt_3$ 2.63 | THF, 150 | 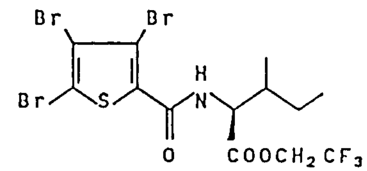 5.23/86-88°C |
| 43 | 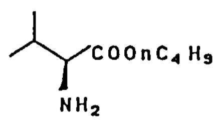 2.73 | 5.0 | $NEt_3$ 2.63 | THF, 150 | 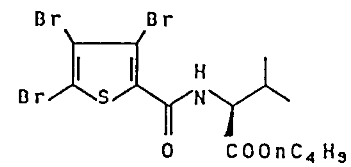 3.3/35-37°C |
| 44 | 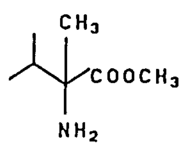 2.73 | 5.75 | $NEt_3$ 3.03 | THF, 100 | 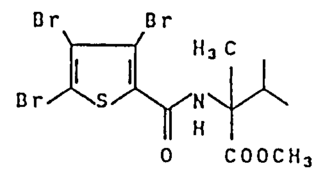 4.94/92-95°C |

| Beispiel | AS-Ester·HCl o. Amid·HCl | 2.3.4.-Tribromthiophen-carbonsäurechlorid [g] | NEt₃ [g] | Lsgm. [ml] | Produkt/Fp |
|---|---|---|---|---|---|
| 45 | 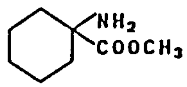 2.01 | 5.0 | $NEt_3$ 2.63 | THF, 150 | 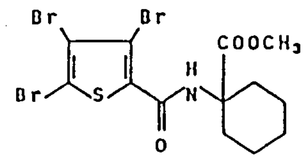 3.75/147-149°C |
| 46 | 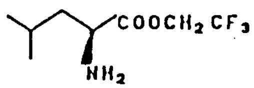 3.29 | 5.0 | $NEt_3$ 2.63 | THF, 150 | 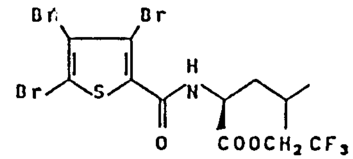 4.71/88-90°C |
| 47 | 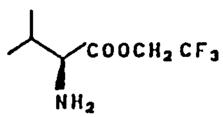 2.36 | 3.83 | $NEt_3$ 2.02 | THF, 150 | 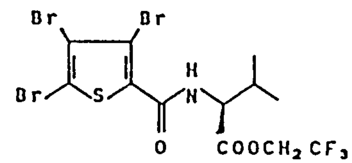 3.54/123-124°C |
| 48 | $H_2N-CH_2-COOCH_3$ 1.64 | 5.0 | $NEt_3$ 2.63 | THF, 150 | 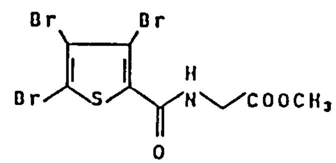 5.23/145-147°C |

| Beispiel | AS-Ester·HCl o. Amid·HCl | 2.3.4.-Tribromthiophen-carbonsäurechlorid [g] | $NEt_3$ [g] | Lsgm. [ml] | Produkt/Fp |
|---|---|---|---|---|---|
| 49 | 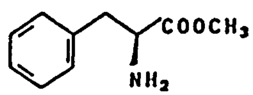 2.25 | 4.0 | $NEt_3$ 2.10 | THF, 150 | 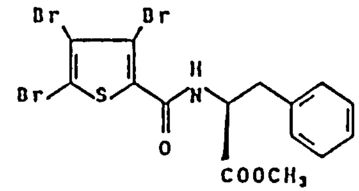 4.16/136-138°C |
| 50 | 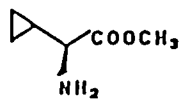 1.9 | 4.39 | $NEt_3$ 2.32 | THF, 100 | 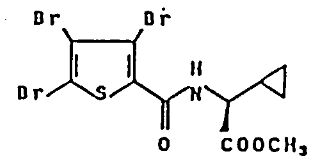 2.61/120-122°C |
| 51 | 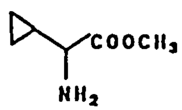 2.5 | 5.5 | $NEt_3$ 3.05 | THF, 70 | 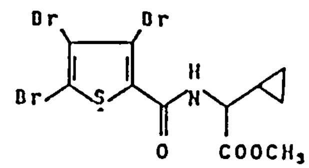 5.35/104-106°C |
| 52 | 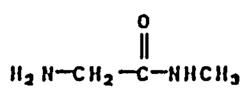 2.43 | 7.5 | $NEt_3$ 3.95 | THF, 150 | 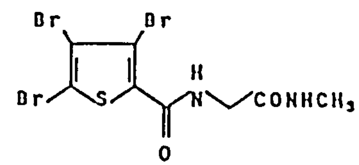 4.28/237-239°C |

| Beispiel | AS-Ester·HCl o. Amid·HCl | 2.3.4.-Tribromthiophen-carbonsäurechlorid [g] | NEt$_3$ [g] | Lsgm. [ml] | Produkt/Fp |
|---|---|---|---|---|---|
| 53 | 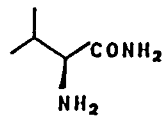 1.06 | 2.93 | NEt$_3$ 1.54 | THF, 150 | 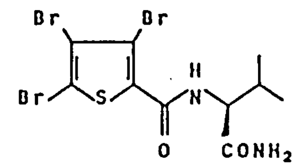 2.56/205-206°C |
| 54 | 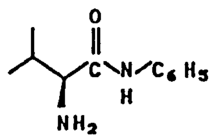 als Acetat 3.29 | 5.0 | NEt$_3$ 2.63 | THF, 150 | 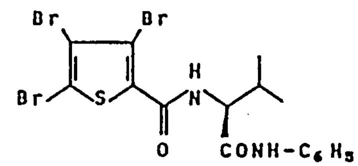 3.87/224-226°C |
| 55 | 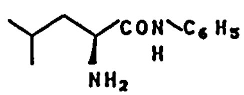 als Acetat 3.47 | 5.0 | NEt$_3$ 2.63 | THF, 150 | 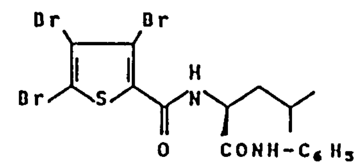 3.0/190-192°C |
| 56 | 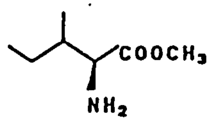 7.08 | 15.0 | NEt$_3$ 7.89 | THF, 150 | 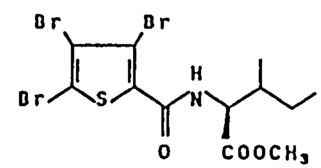 15.1/84-86°C |

| Beispiel | AS-Ester·HCl o. Amid·HCl | 2.3.4.-Tribromthiophen-carbonsäurechlorid [g] | NEt₃ [g] | Lsgm. [ml] | Produkt/Fp |
|---|---|---|---|---|---|
| 57 | 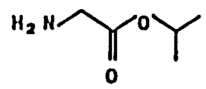 6.0 | 15.0 | $NEt_3$ 7.89 | THF, 150 | 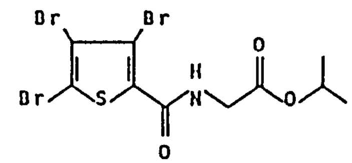 15.46/113-116°C |
| 58 | 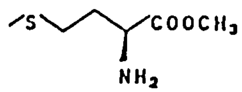 8.68 | 15.0 | $NEt_3$ 7.89 | THF, 150 | 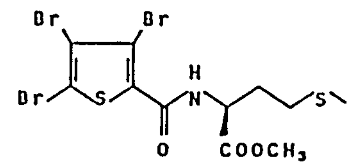 15.0/80-82°C |
| 59 | 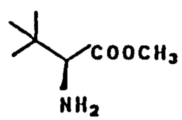 7.00 | 15.0 | $NEt_3$ 7.89 | THF, 150 | 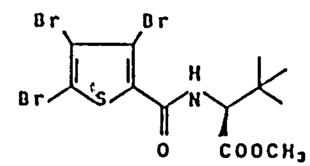 15.9/117-120°C |
| 60 | 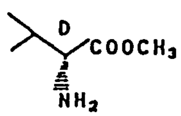 6.54 | 15.0 | $NEt_3$ 7.89 | THF, 150 | 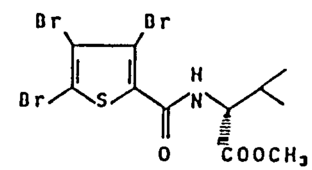 14.9/107-110°C |

| Beispiel | AS-Ester·HCl o. Amid·HCl | 2.3.4.-Tribromthiophen-carbonsäurechlorid [g] | $NEt_3$ [g] | Lsgm. [ml] | Produkt/Fp |
|---|---|---|---|---|---|
| 61 | 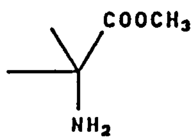 4.02 | 15.0 | $NEt_3$ 7.89 | THF, 150 | 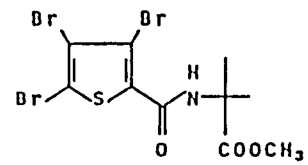 8.91/128-130°C |
| 62 | 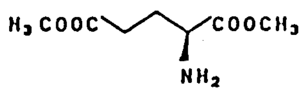 2.76 | 5.0 | $NEt_3$ 2.63 | THF, 150 | 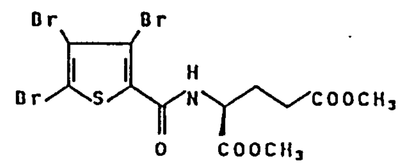 4.9/118-120°C |
| 63 | 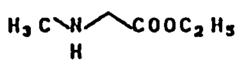 2.0 | 5.0 | $NEt_3$ 2.63 | THF, 150 | 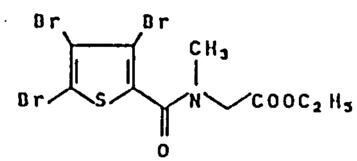 4.56/78-80°C |
| 64 | 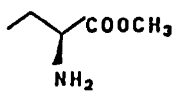 2.0 | 5.0 | $NaHCO_3$ 2.19 | $H_2O$, 100<br>THF, 50 | 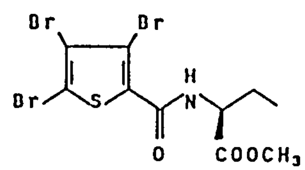 4.97/122-124°C |

| Beispiel | AS-Ester·HCl o. Amid·HCl | 2.3.4.-Tribromthiophen-carbonsäurechlorid [g] | NEt$_3$ [g] | Lsgm. [ml] | Produkt/Fp |
|---|---|---|---|---|---|
| 65 | 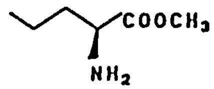 2.19 | 5.0 | NEt$_3$ 2.63 | THF, 150 | 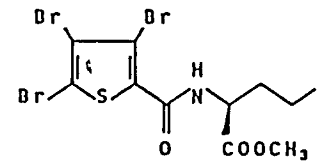 4.75/105-107°C |
| 66 | 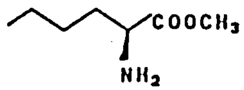 2.37 | 5.0 | NEt$_3$ 2.63 | THF, 150 | 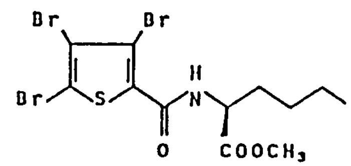 5.03/102-104°C |
| 67 | 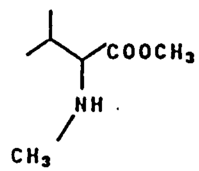 | 5.0 | NEt$_3$ 2.63 | THF, 150 | 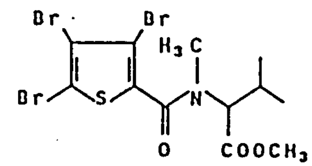 |
| 68 | 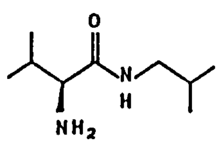 3.98 | 6.59 | $NaHCO_3$ 2.88 | $H_2O$, 100 THF, 50 | 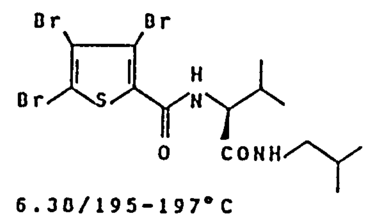 6.38/195-197°C |

| Beispiel | AS-Ester·HCl o. Amid·HCl | 2.3.4.-Tribromthiophen-carbonsäurechlorid [g] | $NEt_3$ [g] | Lsgm. [ml] | Produkt/Fp |
|---|---|---|---|---|---|
| 69 | 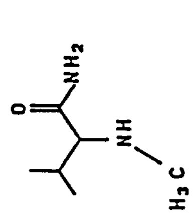 2.17 | 5.0 | $NaHCO_3$ 2.80 | $H_2O$, 100 THF, 50 | 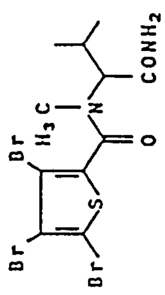 4.55/208-211°C |
| 70 | 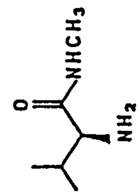 2.0 (als Acetat) | 4.02 | $NaHCO_3$ 2.19 | $H_2O$, 100 THF, 40 | 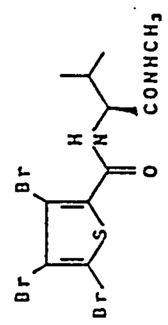 3.44/250-252°C |

Beispiel 71

4.0 g 2.3-Dichlorthiophen-5-carbonsäurechlorid in 50 ml THF werden zu einer Suspension von 3.37 g L-Leucinmethylester•HCl und 3.76 g Triethylamin in 100 ml THF bei 20 °C innerhalb von 3 h zudosiert. Anschließend wird 2 h nachgerührt, von den ausgefallenen Salzen abfiltriert, das Filtrat eingedampft und der

Rückstand mit 2 x 30 ml $H_2O$ ausgerührt, dann mit Petrolether nachgewaschen und bei 40 °C/40 mbar bis zur Gewichtskonstanz getrocknet.

Man erhält 5.25 g (87 % d.Th.) N-(2'.3'-Dichlor-5'-thenoyl)-L-leucinmethylester mit mit einem Schmelzpunkt von 93 - 95 °C.

Beispiel 72

Gemäß Beispiel 71 werden 4.0 g 2.3-Dichlorthiophencarbon-5-säurechlorid mit 3.37 g L-Isoleucinmethylester•HCl umgesetzt.

Nach Aufarbeitung erhält man 3.76 g (62.3 % d.Th.) N-(2'.3'-Dichlor-5'-thenoyl)-L-isoleucinmethylester mit einem Schmelzpunkt von 48 - 50 °C.

Beispiel 73

Gemäß Beispiel 71 werden 4.0 g L-Valinmethylester•HCl mit 5.14 g 2.3-Dichlorthiophen-5-carbonsäurechlorid umgesetzt.

Nach Aufarbeitung erhält man 5.11 g (69 % d.Th.) N-(2'.3'-Dichlor-5'-thenoyl)-L-valinmethylester mit einem Schmelzpunkt von 75 - 76 °C.

Beispiel 74

Gemäß Beispiel 71 werden 2.74 g 2.3-Dichlorthiophen-5-carbonsäurechlorid mit 3.0 g L-Valin-2'.2'.2'-trifluorethylester•HCl umgesetzt.

Nach Aufarbeitung erhält man 4.14 g (86.1 % d.Th.) N-(2'.3'-Dichlor-5'-thenoyl)-L-valin-2''.2''.2''-trifluorethylester mit einem Schmelzpunkt von 53 - 54 °C.

Beispiel 75

Gemäß Beispiel 71 werden 2.81 g 2.3-Dichlorthiophen-5-carbonsäurechlorid mit 3.29 g L-Leucin-2'.2'.2'-trifluorethylester•HCl umgesetzt.

Nach Aufarbeitung erhält man 3.27 g (65 % d.Th.) N-(2'.3'-Dichlor-5'-thenoyl)-L-leucin-2''.2''.2''-trifluorethylester mit einem Schmelzpunkt von 68 - 70 °C.

Beispiel 76

Gemäß Beispiel 71 werden 3.29 g L-Isoleucin-2'.2'.2'-trifluorethylester•HCl mit 2.81 g 2.3-Dichlorthiophen-5-carbonsäurechlorid umgesetzt.

Nach Aufarbeitung erhält man 4.1 g (65.4 % d.Th.) N-(2'.3'-Dichlor-5'-thenoyl)-L-isoleucin-2''.2''.2''-trifluorethylester mit einem Schmelzpunkt von 73 - 75 °C.

Beispiel 77

Gemäß Beispiel 71 werden 5.00 g 2.3.4-Tribromthiophen-carbonsäurechlorid mit 2.89 g L-Hexahydrophenylalaninmethylester•HCl, 2.63 g Triethylamin in 150 ml THF umgesetzt.

Nach Aufarbeitung erhält man 4.2 g (61.2 % d.Th.) N-(2'.3'.4'-Tribromthenoyl)-L-hexahydrophenylalaninmethylester als viskoses Öl.

Beispiel 78

In eine Lösung von 4.45 g NaOH und 4.95 g D.L-Alanin in 60 ml Wasser werden bei 10 °C 12.0 g 2.3-Dichlorthiophen-5-carbonsäurechlorid zugetropft. Nach Reaktionsende wird noch 1 h nachgerührt und anschließend mit konz. HCl auf pH 2 angesäuert. Das ausgeschiedene Öl wird in 100 ml Essigester aufgenommen, 2 mal mit 20 ml Wasser neutral gewaschen gewaschen und nach Trocknen das Lösungsmittel abdestilliert. Der viskose Rückstand kristallisiert nach Versetzen mit 30 ml Diisopropylether. Nach Trocknung bei 40 °C/50 mbar bis zur Gewichtskonstanz erhält man 14.0 g (99.6 % d.Th.) N-(2'.3'-Dichlor-5'-thenoyl)-D.L-alanin mit einem Schmelzpunkt von 195 - 197 °C.

Beispiel 79

Gemäß Beispiel 78 werden 12.0 g 2.3-Dichlorthiophen-5-carbonsäurechlorid mit 4.95 g L-Alanin umgesetzt.

Nach Aufarbeitung erhält man 13.75 g (98.1 % d.Th.) N-(2'.3'-Dichlor-5'-thenoyl)-L-alanin mit einem Schmelzpunkt von 198 - 202 °C.

Beispiel 80

Gemäß Beispiel 78 werden 12.0 g 2.3-Dichlorthiophen-5-carbonsäurechlorid mit 4.95 g D-Alanin umgesetzt.

Nach Aufarbeitung erhält man 13.0 g (92.6 % d.Th.) N-(2'.3'-Dichlor-5'-thenoyl)-D-alanin mit einem Schmelzpunkt von 198 - 200 °C.

Beispiel 81

Gemäß Beispiel 78 werden 10.8 g 2.3-Dichlorthiophen-5-carbonsäurechlorid mit 5.86 g D.L-Valin und 4.0 g NaOH in 60 ml Wasser umgesetzt.

Nach Aufarbeitung erhält man 10.0 g (67.6 % d.Th.) N-(2'.3'-Dichlor-5'-thenoyl)-D.L-valin mit einem Schmelzpunkt von 188 - -190 °C.

Beispiel 82

Gemäß Beispiel 78 werden 12.0 g 2.3-Dichlorthiophen-5-carbonsäurechlorid mit 5.73 g D.L-α-Aminobuttersäure und 4.45 g NaOH in 60 ml Wasser umgesetzt.

Nach Aufarbeitung erhält man 13.47 g (85.7 % d.Th.) N-(2'.3'-Dichlor-5'-thenoyl)-D.L-α-aminobuttersäure.

Beispiel 83

12.0 g 2.3-Dichlorthiophen-5-carbonsäurechlorid werden gemäß Beispiel 78 mit 5.73 g α-Aminoisobuttersäure und 4.45 g NaOH in 60 ml $H_2O$ umgesetzt.

Nach Aufarbeitung mit Diethylether erhält man 7.38 g (47 % d.Th.) N-(2'.3'-Dichlor-5'-thenoyl)-α-aminoisobuttersäure mit einem Schmelzpunkt von 217 - 220 °C.

Beispiel 84

10.8 g 2.3-Dichlorthiophen-5-carbonsäurechlorid werden gemäß Beispiel 78 mit 7.4 g D.L-Methionin und 4.0 g NaOH in 50 ml $H_2O$ umgesetzt.

Nach Aufarbeitung erhält man direkt 16.0 g (97.5 % d.Th.) N-(2'.3'-Dichlor-5'-thenoyl)-D.L-methionin mit einem Schmelzpunkt von 193 - 195 °C.

Beispiel 85

10.8 g 2.3-Dichlorthiophen-5-carbonsäurechlorid werden gemäß Beispiel 78 mit 3.8 g Glycin und 4.0 g NaOH in 50 ml $H_2O$ umgesetzt.

Nach Aufarbeitung und Umkristallisation aus Essigsäureethylester erhält man 6.2 g (48.8 % d.Th) N-(2'.3'-Dichlor-5'-thenoyl)-glycin mit einem Schmelzpunkt von 212 - 214 °C.

Beispiel 86

10.8 g 2.3-Dichlorthiophen-5-carbonsäurechlorid werden gemäß Beispiel 78 mit 7.36 g L-Glutaminsäure und 6.0 g NaOH in 60 ml $H_2O$ umgesetzt.

Nach Aufarbeitung erhält man 9.82 g (60.2 % d.Th.) N-(2'.3'-Dichlor-5'-thenoyl)-L-glutaminsäure mit einem Schmelzpunkt von 150 - 157 °C.

Beispiel 87

10.8 g 2.3-Dichlorthiophen-5-carbonsäurechlorid werden gemäß Beispiel 78 mit 6.56 g L-Isoleucin und 8.4 g $NaHCO_3$ in 60 ml $H_2O$ umgesetzt.

Nach Aufarbeitung erhält man 6.91 g (44.5 % d.Th.) N-(2'.3'-Dichlor-5'-thenoyl)-L-isoleucin mit einem Schmelzpunkt von 130 - 135 °C.

Beispiel 88

10.24 g 2.3-Dichlorthiophen-5-carbonsäurechlorid werden gemäß Beispiel 78 mit 6.32 g L-Thiazolidin-4-carbonsäure und 7.98 g $NaHCO_3$ in 100 ml $H_2O$ umgesetzt.

Nach Aufarbeitung erhält man 4.0 g (27.3 % d.Th.) N-(2'.3'-Dichlor-5'-thenoyl)-L-thiazolidin-4-carbonsäure mit einem Schmelzpunkt von 195 - 200 °C.

Beispiel 89

4.5 g D.L-Alanin werden zusammen mit 4.0 g NaOH in 40 ml $H_2O$ gelöst. Zu dieser Lösung wird bei 50 °C eine Lösung von 10.8 g 2.3-Dibromthiophen-5-carbonsäurechlorid in 10 ml Toluol zugetropft und 1 Stunde nachreagieren gelassen. Nach Phasentrennung wird die wäßrige Phase mit konz. HCl auf pH 2 angesäuert und das sich abscheidende Öl nach kurzem Stehenlassen kristallisiert. Der Feststoff wird abgesaugt, mit $H_2O$ neutral gewaschen und bei 50 °C/50 mbar bis zur Gewichtskonstanz getrocknet.

Man erhält 12.1 g (96.8 % d.Th.) N-(2'.3'-Dibrom-5'-thenoyl)-D.L-alanin mit einem Schmelzpunkt von 205 - 208 °C.

Beispiel 90

15.3 g 2.3.4-Tribromthiophencarbonsäurechlorid in 60 ml Dioxan werden gemäß Beispiel 89 mit 34.56 g L-Alanin und 3.20 g NaOH in 60 ml $H_2O$ umgesetzt.

Nach Aufarbeitung erhält man 10.5 g (60.1 % d.Th.) N-(2'.3'.4'-Tribromthenoyl)-L-alanin mit einem Schmelzpunkt von 175 - 179 °C.

Beispiel 91

15.3 g 2.3.4-Tribromthiophencarbonsäurechlorid in 60 ml Dioxan werden gemäß Beispiel 89 mit 4.69 g g L-Valin und 3.20 g NaOH in 60 ml $H_2O$ umgesetzt.

Nach Aufarbeitung erhält man 11.7 g (63.1 % d.Th.) N-(2'.3'.4'-Tribromthenoyl)-L-valin mit einem Schmelzpunkt von 137 - 140 °C.

Beispiel 92

10 g 2.3.4-Trichlorthiophencarbonsäurechlorid in 50 ml THF werden gemäß Beispiel 89 mit 3.56 g L-Alanin und 3.20 g NaOH in 60 ml $H_2O$ umgesetzt.

Nach Aufarbeitung erhält man 10.2 g (84.5 % d.Th.) N-(2'.3'.4'-Trichlorthenoyl)-L-alanin mit einem Schmelzpunkt von 160 - 164 °C.

Beispiel 93

10 g 2.3.4-Trichlorthiophencarbonsäurechlorid in 50 ml THF werden gemäß Beispiel 89 mit 4.69 g L-Valin und 3.20 g NaOH in 60 ml $H_2O$ umgesetzt.

Nach Aufarbeitung erhält man 11.9 g (90 % d.Th.) N-(2'.3'.4'-Trichlorthenoyl)-L-valin mit einem Schmelzpunkt von 120 - 123 °C.

Beispiel 94

Gemäß Beispiel 78 werden 10.8 g 2.3-Dichlorthiophen-5-carbonsäurechlorid mit 6.56 g L-Leucin und 8.4 g $NaHCO_3$ in 60 ml wasser umgesetzt.

Nach Aufarbeitung erhält man 9.11 g (61.1 % d.Th.) N-(2'.3'-Dichlor-5'-thenoyl)-L-leucin mit einem Schmelzpunkt von 125 - 135 °C.

Beispiel 95

Gemäß Beispiel 40 werden 2.01 g L-Valin, 2.27 g KOH und 5.23 g 2.3-Dibromthiophen-5-carbonsäurechlorid umgesetzt.

Nach Aufarbeitung erhält man 5.3 g (80.0 % d.Th.) N-(2'.3'-Dibrom-5'-thenoyl)-L-valin mit einem Schmelzpunkt von 169 - 173 °C.

Beispiel 96

10.0 g 2.3.4-Trichlorthiophencarbonsäurechlorid in 50 ml THF werden zu einer Lösung von 7.62 g L-Valinethylester•HCl in 50 ml Pyridin bei 20 °C innerhalb von 2 h zudosiert. Anschließend wird 2 h nachgerührt, die Lösungsmittel eingedampft und der Rückstand 2 x mit je 30 ml Wasser ausgerührt. Die dabei ausfallenden Kristalle werden abfiltriert, mit weiterem Wasser neutral gewaschen und bei 50 °C/40 mbar bis zur Gewichtskonstanz getrocknet.

Man erhält 11.35 g (79.1 % d.Th.) N-(2'.3'.4-Trichlorthenoyl)-L-valinethylester mit einem Schmelzpunkt von 66 - 68 °C.

Beispiel 97

Gemäß Beispiel 96 werden 10 g 2.3.4-Trichlorthiophencarbonsäurechlorid mit 6.71 g L-Valinmethylester•HCl umgesetzt.

Nach Aufarbeitung erhält man 9.9 g (71.7 % d.Th.) N-(2'.3'.4'-Trichlorthenoyl)-L-valinmethylester mit einem Schmelzpunkt von 47 - 50 °C.

Beispiel 98

Gemäß Beispiel 58 werden 15 g 2.3.4-Tribromthiophencarbonsäurechlorid mit 8.68 g L-Methioninethylester•HCl umgesetzt.

Nach Aufarbeitung erhält man 15.0 g (73.4 % d.Th) N-(2'.3'.4'-Tribromthenoyl)-L-methioninethylester mit einem Schmelzpunkt von 80 - 82 °C.

Beispiel 99

Gemäß Beispiel 58 werden 15.3 g 2.3.4-Tribromthiophencarbonsäurechlorid mit 5.58 g L-Alaninethylester•HCl umgesetzt.

Nach Aufarbeitung mit 4.6 g (25.5 % d.Th.) N-(2'.3'.4'-Tribromthenoyl)-L-alaninethylester mit einem Schmelzpunkt von 112 - 114 °C.

Beispiel 100

Gemäß Beispiel 71 werden 15.0 g 2.3-Dibromthiophen-5-carbonsäurechlorid mit 9.6 g L-Methioninethylester•HCl umgesetzt. Nach Aufarbeitung erhält man 14.5 g (76% d.Th.) N-(2'.3'-Dibrom-5'-thenoyl)-L-methioninethylester als viskoses Öl.

BIOLOGISCHE BEISPIELE

Beispiel 1: Wirkung gegen Colletotrichum lagenarium auf Gurkenuflanzen (Cucumis sativus L)

a) Gurkenpflanzen werden nach 2-wöchiger Anzucht mit einer aus Spritzpulver des Wirkstoffes hergestellten Spritzbrühe besprüht (Konzentration: 200 ppm).

Nach 48 Stunden werden die Pflanzen mit einer Sporensuspension ($1.5 \cdot 10^3$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C bis 23 °C weitergeführt.

Die Beurteilung der Schutzwirkung erfolgt aufgrund des Pilzbefalls 7-8 Tage nach der Infektion.

b) Gurkenplanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen).

Nach 48 Stunden werden die Pflanzen mit einer Sporensuspension (1.5 • $10^3$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C weitergeführt.

Die Beurteilung der Schutzwirkung erfolgt aufgrund des Pilzbefalls 7-8 Tage nach der Infektion.

Unbehandelte aber infizierte Kontrollpflanzen weisen in den Tests a) und b) einen Pilzbefall von 100 % auf.

Verbindungen gemäß der vorliegenden Erfindung bewirken einen guten Schutz gegen Colletotrichum lagenarium. So bleiben Pflanzen, die z.B. mit den Verbindungen der Beispiele Nr. 4, 11, 13, 84, 85, 89, 91, 92, 93 und 96 behandelt wurden, weitgehend frei von Colletotrichum (Befall 0 - 20 %).

Beispiel 2: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt.

Die Beurteilung der Rostpustelnentwicklung erfolgt 12 Tage nach der Infektion.

b) Zu Weizenpflanzen werden 5 Tage nach Aussaat eine aus Spritzpulver des Wirkstoffes hergestellten Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

Unbehandelte aber infizierte Kontrollpflanzen weisen in den Tests a) und b) einen Pilzbefall von 100 % auf.

Verbindungen gemäß der vorliegenden Erfindung bewirken einen guten Schutz gegen Puccinia graminis So bleiben Pflanzen, die z.B. mit den Verbindungen der Beispiele Nr. 9, 71, 72, 75, 76 und 85 behandelt wurden, weitgehend frei von Puccinia (Befall 0 - 20 %).

Beispiel 3: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20 °C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, daß die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100% relativer Luftfeuchtigkeit und 20 °C.

Unbehandelte aber infizierte Kontrollpflanzen weisen in den Tests a) und b) einen Pilzbefall von 100 % auf.

Verbindungen gemäß der vorliegenden Erfindung bewirken einen guten Schutz gegen Phytophthora infestans. So bleiben Pflanzen, die z.B. mit den Verbindungen der Beispiele Nr. 1, 3, 4, 10, 11, 13, 27, 37, 48, 50, 73, 79, 80, 81, 82, 83, 85, 86, 87, 90, 92, 96, 97 und 99 behandelt wurden, weitgehend frei von Phytophthora (Befall 0 - 20 %).

Beispiel 4: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert.

Nach 5 Tagen Inkubation bei 95-100 % relativer Luftfeuchtigkeit und 24 °C wird der Pilzbefall beurteilt.

b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, daß die untersten Stengelteile der Reispflanzen im Wasser stehen. Nach 96 Stunden werden die behandelten Reispflanzen mit einer Konidiensuspension des Pilzes infiziert.

Nach einer Inkubation der infizierten Pflanzen während 5 Tagen bei 90-100% relativer Luftfeuchtigkeit und ca. 24 °C wird der Pilzbefall beurteilt.

Unbehandelte aber infizlerte Kontrollpflanzen weisen in den Tests a) und b) einen Pilzbefall von 100 % auf.

Verbindungen gemäß der vorliegenden Erfindung bewirken einen guten Schutz gegen Pyricularia oryzae. So bleiben Pflanzen, die z.B. mit den Verbindungen der Beispiele Nr. 3, 4, 13, 27, 49, 73, 76, 78, 79, 80, 83 und 87 behandelt wurden, weitgehend frei von Pyricularia (Befall 0 - 20 %).

Beispiel 5: Wirkung gegen Pseudomonas tomato an Tomatenpflanzen

a) Tomatenpflanzen werden nach 3-wöchiger Anzucht mit einer aus Spritzpulver des Wirkstoffes hergestellten Spritzbrühe durch Blattapplikation behandelt (Konzentration: 200 ppm). Nach 3,5 Wochen werden die Pflanzen mit einer Bakteriensuspension ($10^8$ Bakterien/ml) inokuliert und für 5 Tage bei hoher Luftfeuchtigkeit und bei einer Temperatur von 25 °C inkubiert. Die Beurteilung der Schutzwirkung erfolgt aufgrund des Bakterienbefalls 7-8 Tage nach Inokulation.

b) Tomatenpflanzen werden nach 3-wöchiger Anzucht mit einer aus Spritzpulver des Wirkstoffes hergestellten Spritzbrühe durch Bodenapplikation behandelt (Konzentration: 60 ppm bezogen auf das Bodenvolumen).

Nach 3,5 Wochen werden die Pflanzen mit einer Bakteriensuspension ($10^8$ Bakterien/ml) inokuliert und für 6 Tage bei hoher Luftfeuchtigkeit und bei einer Temperatur von 25 °C inkubiert.

Die Beurteilung der Schutzwirkung erfolgt aufgrund des Bakterienbefalls 7-8 Tage nach Inokulation.

Unbehandelte aber infizierte Kontrollpflanzen weisen in den Tests a) und b) einen Pilzbefall von 100 % auf.

Verbindungen gemäß der vorliegenden Erfindung bewirken einen guten Schutz gegen Pseudomonas tomato. So bleiben Pflanzen, die z.B. mit den Verbindungen der Beispiele Nr. 1, 6, 16, 18, 35, 38, 48, 51, 73, 98 und 100 behandelt wurden, weitgehend frei von Pseudomonas (Befall 0 - 20 %).

Beispiel 6: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelags werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmaß dieser Krankheitssymptome dient zur Beurteilung der residualen Wirksamkeit der Prüfsubstanz.

b) Reispflanzen der Sorte "Calora" oder "S6" werden nach 3-wöchiger Anzucht im Gewächshaus mit der Prüfsubstanz in Form einer Spritzbrühe (0,006 % Aktivsubstanz bezogen auf das Erdvolumen). Drei Tage nach dieser Behandlung werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmaß dieser Krankheitssymptome dient zur Beurteilung der systemischen Wirksamkeit der Prüfsubstanz.

Unbehandelte aber infizierte Kontrollpflanzen weisen in den Tests a) und b) einen Pilzbefall von 100 % auf.

Verbindungen gemäß der vorliegenden Erfindung bewirken einen guten Schutz gegen Xanthomonas oryzae. So bleiben Pflanzen, die z.B. mit den Verbindungen der Beispiele Nr. 1, 4, 16, 18, 19, 27, 35, 38, 51, 73, 84 und 100 behandelt wurden, weitgehend frei von Xanthomonas (Befall 0 - 20 %).

Beispiel 7: Wirkung gegen Xanthomonas versicatoria auf Paprika (Capsicum annuum)

a) Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelags werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas versicatoria infiziert. Nach 6-tägiger Inkubation bilden sich bei Befall auf den Blättern runde, anfangs wäßrige, später nekrotische, aufgehellte Flecken.

Das Ausmaß dieser Krankheitssymptome dient zur Beurteilung der residualen Wirksamkeit der Prüfsubstanz.

b) Paprikapflanzen der Sorte "California Wonder" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas versicatoria infiziert. Nach 6-tägiger Inkubation bilden sich bei Befall auf den Blättern runde, anfangs wäßrlge, später nekrotische, aufgehellte Flecken.

Das Ausmaß dieser Flecken dient zur Beurteilung der systemischen Wirksamkeit der Prüfsubstanz.

Unbehandelte aber infizierte Kontrollpflanzen weisen in den Tests a) und b) einen Pilzbefall von 100 % auf.

Verbindungen gemäß der vorliegenden Erfindung bewirken einen guten Schutz gegen Xanthomonas versicatoria. So bleiben Pflanzen, die z.B. mit den Verbindungen der Beispiele Nr. 1, 4, 6, 7, 16, 18, 19, 27, 28, 31, 35, 38, 56, 73 und 84 behandelt wurden, weitgehend frei von Xanthomonas (Befall 0 - 20 %).

## Patentansprüche

## Patentansprüche für folgende Vertragsstaaten : AT, BE, CH, DE, DK, FR, GB, GR, IT, LI, NL, SE

**1.** Verbindungen der allg. Formel I

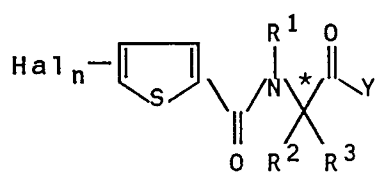

I

wobei Hal Chlor oder Brom, n die Zahl 2 oder 3,

* bei unterschiedlichen Resten $R^2$, und $R^3$ entweder ein Enantiomeres oder ein Racemat bedeutet,

$R^1$ für Wasserstoff, einen geradkettigen oder verzweigten Alkyl-Rest mit 1-4 C-Atomen,

$R^2$ für Wasserstoff, einen geradkettigen Alkyl-Rest mit 1-16 C-Atomen bzw. verzweigtkettigen Alkyl-Rest mit 3-16 C-Atomen, der gegebenenfalls durch Hydroxy-, Carboxy-, $C_{1-6}$-Alkoxy- oder $C_{1-6}$-Alkoxycarbonyl, Mercapto- oder $C_{1-6}$-Alkylmercapto- oder Benzylmercapto substituiert sein kann, einen Aryl-Rest mit 6-10 C-Atomen bzw. Aralkyl-Rest mit 7-16 C-Atomen, wobei das Ringsystem gegebenenfalls mit 1-3 Fluor-, Chlor-, oder Brom-Atomen substituiert sein kann, Cycloalkyl-Rest mit 3-8 C-Atomen, Cycloalkyl-methyl-Rest mit insgesamt 4-9 C-Atomen,

$R^3$ für Wasserstoff, einen Alkyl-Rest mit 1-4 C-Atomen, einen Alkenyl- oder Alkinyl-Rest mit 2-4 C-Atomen stehen oder gegebenenfalls $R^1$ und $R^3$ oder $R^2$ und $R^3$ zusammen eine Alkylen-Brücke mit 2-8 C-Atomen bilden können bei der gegebenenfalls eine $CH_2$-Einheiten durch $NR_1$, O oder S substituiert sein kann,

Y OH, $OR_4$ oder

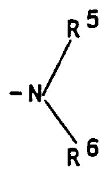

bedeutet, wobei

$R^4$ für einen Alkyl-Rest mit 1-4 C-Atomen steht, dessen H-Atome gegebenenfalls ganz oder teilweise gegen Halogenatome ausgetauscht sind,

$R^5$ Wasserstoff, einen Alkyl-Rest mit 1-6 C-Atomen, Hydroxy- oder einen Alkoxy-Rest mit 1-6 C-Atomen,

$R^6$ Wasserstoff, einen Alkyl-Rest mit 1-6 C-Atomen, einen Phenyl-Rest oder einen Cycloalkyl-Rest mit 3-8 C-Atomen bedeutet oder gegebenenfalls $R^5$ und $R^6$ unter Einbeziehung des Stickstoffs-Atoms und gegebenenfalls eines weiterer Heteroatoms einen 4-8-gliedrigen Ring bilden kann.

**2.** Verbindungen gemäß Anspruch 1, dadurch gekennzeichnet, daß

$R^1$ Wasserstoff oder einen Alkyl-Rest mit 1-3 C-Atomen bedeutet,

$R^2$ Wasserstoff, einen Alkyl-Rest mit 1-6 C-Atomen darstellt, der unsubstituiert oder durch einen Alkoxy-carbonyl-Rest mit 1-6 C-Atomen substituiert sein kann, ein Phenyl-, Aralkyl- mit 7 oder 8 C-Atomen oder Cycloalkyl-Rest mit 3-7 C-Atomen ist,

$R^3$ Wasserstoff, oder Alkyl-Rest mit 1-4 C-Atomen ist oder $R^2$ und $R^3$ zusammen eine Alkylenbrücke mit 2 C-Atomen bilden und

Y OH, $OR_4$ oder $NR_5R_6$ ist, worin

$R^4$ einen Alkyl-Rest mit 1-4 C-Atomen bedeutet, dessen Wasserstoff ganz oder teilweise gegen Fluor ausgetauscht sein kann, und

$R^5$ und $R^6$ unabhängig voneinander Wasserstoff oder Alkyl- mit 1-4 C-Atomen oder Alkoxy- mit 1-4 C-Atomen bedeutet oder worin $R^5$ und $R^6$ zusammen mit dem N-Atom einen 5-6-gliedrigen Ring bilden, der kein oder ein weiteres N oder O enthält.

**3.** Verbindungen gemäß Anspruch 1 und 2, dadurch gekennzeichnet, daß Hal Brom, n die Zahl 2 und $R^1$ Wasserstoff bedeuten.

**4.** Verbindungen gemäß Anspruch 1-3, dadurch gekennzeichnet, daß

$R^2$ Wasserstoff oder einen Alkyl-Rest mit 1-4 C-Atomen bedeutet, der unsubstituiert oder durch einen Alkoxycarbonyl-Rest mit 1-2 C-Atomen substituiert sein kann,

$R^3$ Wasserstoff-, Methyl-, oder Ethyl ist, und

Y OH, $OR^4$- oder $NR_5R_6$ bedeutet, worin $R^4$ einen Alkyl-Rest mit 1-4 C-Atomen und $R^5$ und $R^6$ unabhängig voneinander Wasserstoff oder einen Alkyl-Rest mit 1-4 C-Atomen bedeuten.

**5.** Verbindungen gemäß Anspruch 1-4, dadurch gekennzeichnet, daß $Hal_n$ 2 Bromatome in Position 2' und 3' des Thiophenrings bedeuten.

**6.** Verfahren zur Herstellung von Verbindungen gemäß Anspruch 1-5, dadurch gekennzeichnet, daß man ein Säurehalogenid der allgemeinen Formel II

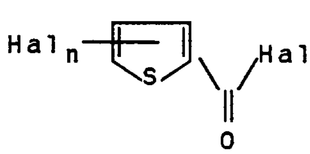

II

wobei Hal = Chlor oder Brom und n die Zahl 2 oder 3 bedeutet, mit einer Aminokomponente der allgemeinen Formel III

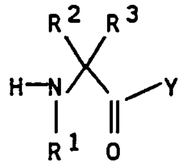

III

in der $R^1$, $R^2$, $R^3$ und Y die im Anspruch 1-5 angegebenen Bedeutungen haben, in Gegenwart einer Base umsetzt.

**7.** Mittel zur Bekämpfung von pflanzenpathogenen Keimen und zur Verhütung von Krankheitsbefall an Pflanzen, dadurch gekennzeichnet, daß es als aktive Komponente mindestens eine Verbindung gemäß Anspruch 1 zusammen mit einem geeigneten Trägermaterial enthält.

**8.** Verfahren zur Herstellung von Mitteln zur Bekämpfung von pflanzenpathogenen Keimen und zur Verhütung von Krankheitsbefall an Pflanzen, dadurch gekennzeichnet, daß es neben einem geeigneten Trägermaterial als aktive Komponente mindestens eine Verbindung gemäß Anspruch 1 enthält.

**9.** Verwendung von Verbindungen der Formel 1 gemäß Anspruch 1 zur Bekämpfung von pflanzenpathogenen Keimen und zur Verhütung von Krankheitsbefall an Pflanzen.

**10.** Verfahren zum Schützen von Pflanzen vor Krankheitsbefall, dadurch gekennzeichnet, daß die Pflanze ihre Pflanzenteile oder der Ort ihres Wachstums mit einer wirksamen Menge einer Verbindung der Formel 1 gemäß Anspruch 1 behandelt wird.

**Patentansprüche für folgenden Vertragsstaat : ES**

**1.** Verfahren zur Herstellung von Verbindungen der allgemeinen Formel I

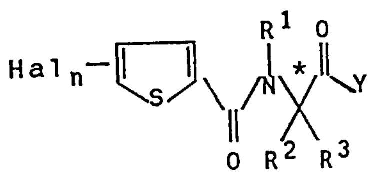

I.

wobei Hal Chlor oder Brom, n die Zahl 2 oder 3,

| | |
|---|---|
| * | bei unterschiedlichen Resten $R^2$, und $R^3$ entweder ein Enantiomeres oder ein Racemat bedeutet, |
| $R^1$ | für Wasserstoff, einen geradkettigen oder verzweigten Alkyl-Rest mit 1-4 C-Atomen, |
| $R^2$ | für Wasserstoff, einen geradkettigen Alkyl-Rest mit 1-16 C-Atomen bzw. verzweigtkettigen Alkyl-Rest mit 3-16 C-Atomen, der gegebenenfalls durch Hydroxy-, Carboxy-, $C_{1-6}$-Alkoxy- oder $C_{1-6}$-Alkoxylcarbonyl, Mercapto- oder $C_{1-6}$-Alkylmercapto- oder Benzylmercapto substituiert sein kann, einen Aryl-Rest mit 6-10 C-Atomen bzw. Aralkyl-Rest mit 7-16 C-Atomen, wobei das Ringsystem gegebenenfalls mit 1-3 Fluor-, Chlor-, oder Brom-Atomen substituiert sein kann, Cycloalkyl-Rest mit 3-8 C-Atomen, Cycloalkyl-methyl-Rest mit insgesamt 4-9 C-Atomen, |
| $R^3$ | für Wasserstoff, einen Alkyl-Rest mit 1-4 C-Atomen, einen Alkenyl- oder Alkinyl-Rest mit 2-4 C-Atomen stehen oder gegebenenfalls $R^1$ und $R^3$ oder $R^2$ und $R^3$ zusammen eine Alkylen-Brücke mit 2-8 C-Atomen bilden können bei der gegebenenfalls eine $CH_2$-Einheit durch $NR_1$, O oder S substituiert sein kann, |
| Y | OH, $OR_4$ oder |

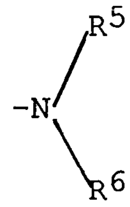

bedeutet, wobei

$R^4$ für einen Alkyl-Rest mit 1-4 C-Atomen steht, dessen H-Atome gegebenenfalls ganz oder teilweise gegen Halogenatome ausgetauscht sind,

$R^5$ Wasserstoff, einen Alkyl-Rest mit 1-6 C-Atomen, Hydroxy- oder einen Alkoxy-Rest mit 1-6 C-Atomen,

$R^6$ Wasserstoff, einen Alkyl-Rest mit 1-6 C-Atomen, einen Phenyl-Rest oder einen Cycloalkyl-Rest mit 3-8 C-Atomen bedeutet und gegebenenfalls $R^5$ und $R^6$ unter Einbeziehung des Stickstoff-Atoms und gegebenenfalls eines weiteren Heteroatoms einen 4-8-gliedrigen Ring bilden kann, dadurch gekennzeichnet, daß man ein Säurehalogenid der allgemeinen Formel II

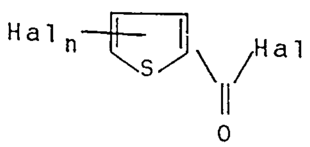

II

wobei Hal = Chlor oder Brom und n die Zahl 2 oder 3 bedeutet, mit einer Aminokomponente der allgemeinen Formel III

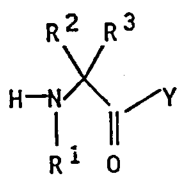

III

in der $R^1$, $R^2$, $R^3$ und Y die im Anspruch 1-5 angegebenen Bedeutungen haben, in Gegenwart einer Base umsetzt.

**2.** Herstellverfahren gemäß Anspruch 1, dadurch gekennzeichnet, daß

$R^1$ Wasserstoff oder einen Alkyl-Rest mit 1-3 C-Atomen bedeutet,

$R^2$ Wasserstoff, einen Alkyl-Rest mit 1-6 C-Atomen darstellt, der unsubstituiert oder durch einen Alkoxy-carbonyl-Rest mit 1-6 C-Atomen substituiert sein kann, ein Phenyl, Aralkyl- mit 7 oder 8 C-Atomen oder Cycloalkyl-Rest mit 3-7 C-Atomen ist,

$R^3$ Wasserstoff, oder Alkyl-Rest mit 1-4 C-Atomen ist oder $R^2$ und $R^3$ zusammen eine Alkylenbrücke mit 2 C-Atomen bilden und

Y OH, $OR_4$ oder $NR_5R_6$ ist, worin

$R^4$ einen Alkyl-Rest mit 1-4 C-Atomen bedeutet, dessen Wasserstoff ganz oder teilweise gegen Fluor ausgetauscht sein kann, und

$R^5$ und $R^6$ unabhängig voneinander Wasserstoff oder Alkyl- mit 1-4 C-Atomen oder Alkoxy- mit 1-4 C-Atomen bedeutet oder worin $R^5$ und $R^6$ zusammen mit dem N-Atom einen 5-6-gliedrigen Ring bilden , der kein oder ein weiteres N oder O enthält.

**3.** Herstellverfahren gemäß Anspruch 1 und 2, dadurch gekennzeichnet, daß Hal Brom, n die Zahl 2 und $R^1$ Wasserstoff bedeuten.

4. Herstellverfahren gemäß Anspruch 1-3, dadurch gekennzeichnet, daß
   - $R^2$ Wasserstoff oder einen Alkyl-Rest mit 1-4 C-Atomen bedeutet, der unsubstituiert oder durch einen Alkoxycarbonyl-Rest mit 1-2 C-Atomen substituiert sein kann,
   - $R^3$ Wasserstoff, Methyl-, oder Ethyl ist, und
   - Y OH, $OR^4$- oder $NR_5R_6$ bedeutet, worin $R^4$ einen Alkyl-Rest mit 1-4 C-Atomen und $R^5$ und $R^6$ unabhängig voneinander Wasserstoff oder einen Alkyl-Rest mit 1-4 C-Atomen bedeuten.

5. Herstellverfahren gemäß Anspruch 1-4, dadurch gekennzeichnet, daß $Hal_n$ 2 Bromatome in Position 2' und 3' des Thiophenrings bedeuten.

6. Verfahren zur Herstellung von Mitteln zur Bekämpfung von pflanzenpathogenen Keimen und zur Verhütung von Krankheitsbefall an Pflanzen, dadurch gekennzeichnet, daß es neben einen geeigneten Trägermaterial als aktive Komponente mindestens eine Verbindung gemäß Anspruch 1 enthält.

7. Verwendung von Verbindungen der Formel I gemäß Anspruch 1 zur Bekämpfung von pflanzenpathogenen Keimen und zur Verhütung von Krankheitsbefall an Pflanzen.

8. Verfahren zum Schützen von Pflanzen vor Krankheitsbefall, dadurch gekennzeichnet, daß die Pflanze, ihre Pflanzenteile oder der Ort ihres Wachstums mit einer wirksamen Menge einer Verbindung der Formel I gemäß Anspruch 1 behandelt wird.

**Claims**
**Claims for the following Contracting States : AT, BE, CH, DE, DK, FR, GB, GR, IT, LI, NL, SE**

1. Compounds of the general formula I

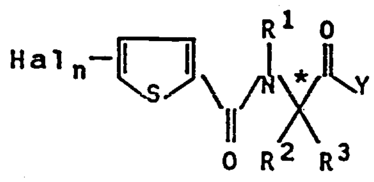

I

wherein Hal signifies chlorine or bromine, n is the number 2 or 3,
   - * signifies, when the radicals $R^2$ and $R^3$ are different, either an enantiomer or a racemate,
   - $R^1$ represents hydrogen, a straight-chain or branched alkyl radical having 1-4 C atoms,
   - $R^2$ represents hydrogen, a straight-chain alkyl radical having 1-16 C atoms or branched-chain alkyl radical having 3-16 C atoms, which optionally can be substituted by hydroxy, carboxy, $C_{1-6}$ alkoxy or $C_{1-6}$ alkoxycarbonyl, mercapto or $C_{1-6}$ alkylmercapto or benzylmercapto, an aryl radical having 6-10 C atoms or aralkyl radical having 7-16 C atoms, wherein the ring system optionally can be substituted by 1-3 fluorine atoms, chlorine atoms or bromine atoms, cycloalkyl radical having 3-8 C atoms, cycloalkyl methyl radical having a total of 4-9 C atoms,
   - $R^3$ represents hydrogen, an alkyl radical having 1-4 C atoms, an alkenyl or alkinyl radical having 2-4 C atoms, or optionally $R^1$ and $R^3$ or $R^2$ and $R^3$ together can form an alkylene bridge having 2-8 C atoms, wherein optionally a $CH_2$ unit can be substituted by $NR_1$, O or S,
   - Y signifies OH, $OR_4$ or

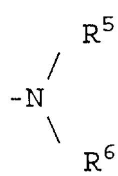

wherein

R⁴ represents an alkyl radical having 1-4 C atoms, the H atoms whereof optionally are completely or partly substituted by halogen atoms,

$R^5$ signifies hydrogen, an alkyl radical having 1-6 C atoms, hydroxy or an alkoxy radical having 1-6 C atoms,

$R^6$ signifies hydrogen, an alkyl radical having 1-6 C atoms, a phenyl radical or a cycloalkyl radical having 3-8 C atoms, or optionally $R^5$ and $R^6$ including the nitrogen atom and optionally another hetero atom can form a 4 to 8-membered ring.

**2.** Compounds according to claim 1, characterised in that

$R^1$ signifies hydrogen or an alkyl radical having 1-3 C atoms,

$R^2$ represents hydrogen, an alkyl radical having 1-6 C atoms, which can be unsubstituted or substituted by an alkoxycarbonyl radical having 1-6 C atoms, or is a phenyl, aralkyl having 7 or 8 C atoms or cycloalkyl radical having 3-7 C atoms,

$R^3$ is hydrogen, or alkyl radical having 1-4 C atoms or $R^2$ and $R^3$ together form an alkylene bridge having 2 C atoms and

Y is OH, $OR_4$ or $NR_5R_6$, wherein

$R^4$ signifies an alkyl radical having 1-4 C atoms, whereof the hydrogen atom can be completely or partly substituted by fluorine and

$R^5$ and $R^6$ independently of one another signify hydrogen or alkyl having 1-4 C atoms or alkoxy having 1-4 C atoms or wherein $R^5$ and $R^6$ together with the N atom form a 5 to 6-membered ring which contains no N or O or another N or O.

**3.** Compounds according to claim 1 or 2, characterised in that Hal signifies bromine, n is the number 2 and $R^1$ signifies hydrogen.

**4.** Compounds according to claims 1 to 3, characterised in that

$R^2$ signifies hydrogen or an alkyl radical having 1-4 C atoms, which can be unsubstituted or substituted by an alkoxycarbonyl radical having 1-2 C atoms,

$R^3$ signifies hydrogen, methyl or ethyl, and

Y signifies OH, $OR^4$ or $NR_5R_6$, wherein $R^4$ signifies an alkyl radical having 1-4 C atoms and $R^5$ and $R^6$ independently of one another signify hydrogen or an alkyl radical having 1-4 C atoms.

**5.** Compounds according to claims 1 to 4, characterised in that $Hal_n$ signifies 2 bromine atoms in the positions 2' and 3' of the thiophene ring.

**6.** Method for the preparation of compounds according to claims 1 to 5, characterised in that an acid halide of the general formula II

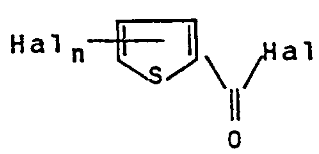

II

wherein Hal is chlorine or bromine and n signifies the number 2 or 3, is reacted with an amino component of the general formula III

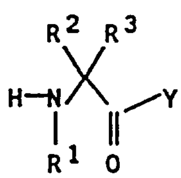

III

wherein $R^1$, $R^2$, $R^3$ and Y have the meanings given in claims 1 to 5, in the presence of a base.

**7.** Agent for the control of plant pathogens and for the prevention of attack by disease on plants, characterised in that it contains as the active component at least one compound according to claim 1 together with a suitable carrier.

**8.** Method for the preparation of agents for the control of plant pathogens and for the prevention of attack by disease on plants, characterised in that it contains, in addition to a suitable carrier, at least one compound according to claim 1 as the active component.

**9.** Use of compounds of the formula I according to claim 1 for the control of plant pathogens and for the prevention of attack by disease on plants.

**10.** Method for the protection of plants from attack by disease, characterised in that the plants, the parts thereof or the site of their growth is treated with an effective quantity of a compound of the formula I according to claim 1.

**Claims for the following Contracting State : ES**

**1.** Process for the preparation of compounds of the general formula I

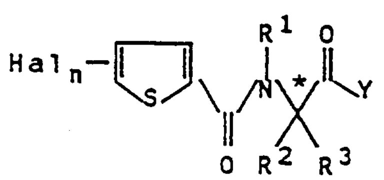

I

wherein Hal signifies chlorine or bromine, n is the number 2 or 3,

- * signifies, when the radicals $R^2$ and $R^3$ are different, either an enantiomer or a racemate,
- $R^1$ represents hydrogen, a straight-chain or branched alkyl radical having 1-4 C atoms,
- $R^2$ represents hydrogen, a straight-chain alkyl radical having 1-16 C atoms or branched-chain alkyl radical having 3-16 C atoms, which optionally can be substituted by hydroxy, carboxy, $C_{1-6}$ alkoxy or $C_{1-6}$ alkoxycarbonyl, mercapto or $C_{1-6}$ alkylmercapto or benzylmercapto, an aryl radical having 6-10 C atoms or aralkyl radical having 7-16 C atoms, wherein the ring system optionally can be substituted by 1-3 fluorine atoms, chlorine atoms or bromine atoms, cycloalkyl radical having 3-8 C atoms, cycloalkyl methyl radical having a total of 4-9 C atoms,
- $R^3$ represents hydrogen, an alkyl radical having 1-4 C atoms, an alkenyl or alkinyl radical having 2-4 C atoms, or optionally $R^1$ and $R^3$ or $R^2$ and $R^3$ together can form an alkylene bridge having 2-8 C atoms, wherein optionally a $CH_2$ unit can be substituted by $NR_1$, O or S,
- Y signifies OH, $OR_4$ or

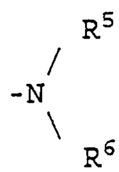

wherein

- $R^4$ represents an alkyl radical having 1-4 C atoms, the H atoms whereof optionally are completely or partly substituted by halogen atoms,
- $R^5$ signifies hydrogen, an alkyl radical having 1-6 C atoms, hydroxy or an alkoxy radical having 1-6 C atoms,
- $R^6$ signifies hydrogen, an alkyl radical having 1-6 C atoms, a phenyl radical or a cycloalkyl radical having 3-8 C atoms, or optionally $R^5$ and $R^6$ including the nitrogen atom and optionally another hetero atom can form a 4 to 8-membered ring

, characterised in that an acid halide of the general formula II

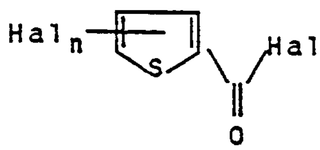

II

wherein Hal is chlorine or bromine and n signifies the number 2 or 3, is reacted with an amino component of the general formula III

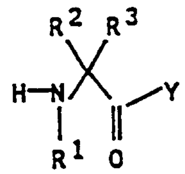

III

wherein $R^1$, $R^2$, $R^3$ and Y have the meanings given in claims 1 to 5, in the presence of a base.

**2.** Process for the preparation according to claim 1, characterised in that
$R^1$ signifies hydrogen or an alkyl radical having 1-3 C atoms,
$R^2$ represents hydrogen, an alkyl radical having 1-6 C atoms, which can be unsubstituted or substituted by an alkoxycarbonyl radical having 1-6 C atoms, or is a phenyl, aralkyl having 7 or 8 C atoms or cycloalkyl radical having 3-7 C atoms,
$R^3$ is hydrogen, or alkyl radical having 1-4 C atoms or
$R^2$ and $R^3$ together form an alkylene bridge having 2 C atoms and
Y is OH, $OR_4$ or $NR_5R_6$, wherein
$R^4$ signifies an alkyl radical having 1-4 C atoms, whereof the hydrogen atom can be completely or partly substituted by fluorine and
$R^5$ and $R^6$ independently of one another signify hydrogen or alkyl having 1-4 C atoms or alkoxy having 1-4 C atoms or wherein $R^5$ and $R^6$ together with the N atom form a 5 to 6-membered ring which contains no N or O or another N or O.

**3.** Process for the preparation according to claim 1 and 2, characterised in that Hal signifies bromine, n is the number 2 and $R^1$ signifies hydrogen.

**4.** Process for the preparation according to claim 1 to 3, characterised in that
$R^2$ signifies hydrogen or an alkyl radical having 1-4 C atoms, which can be unsubstituted or substituted by an alkoxycarbonyl radical having 1-2 C atoms,
$R^3$ signifies hydrogen, methyl or ethyl, and
Y signifies OH, $OR^4$ or $NR_5R_6$, wherein
$R^4$ signifies an alkyl radical having 1-4 C atoms and $R^5$ and $R^6$ independently of one another signify hydrogen or an alkyl radical having 1-4 C atoms.

**5.** Process for the preparation according to claim 1 to 4, characterised in that $Hal_n$ signifies 2 bromine atoms in the positions 2' and 3' of the thiophene ring.

**6.** Method for the preparation of agents for the control of plant pathogens and for the prevention of attack by disease on plants, characterised in that it contains, in addition to a suitable carrier, at least one compound according to claim 1 as the active component.

**7.** Use of compounds of the formula I according to claim 1 for the control of plant pathogens and for the prevention of attack by disease on plants.

8. Method for the protection of plants from attack by disease, characterised in that the plants, the parts thereof or the site of their growth is treated with an effective quantity of a compound of the formula I according to claim 1.

## Revendications

**Revendications pour les Etats contractants suivants : AT, BE, CH, DE, DK, FR, GB, GR, IT, LI, NL, SE**

1. Composés de formule générale I

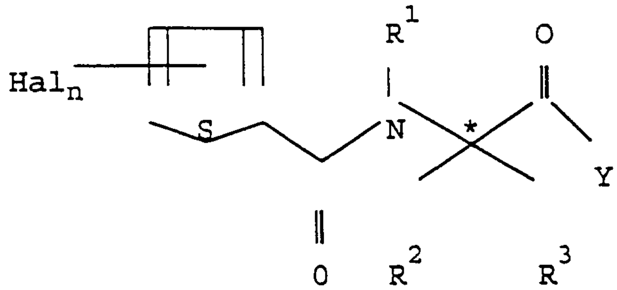

I

dans laquelle Hal représente le chlore ou le brome, n le nombre 2 ou 3,

* représente pour des restes $R^2$ et $R^3$ différents, soit un énantiomère, soit un racémate,

$R^1$ représente un hydrogène, un reste alkyle linéaire ou ramifié de 1-4 atomes de C,

$R^2$ est un hydrogène, un reste alkyle à chaîne linéaire de 1-16 atomes de C ou un reste alkyle à chaîne ramifiée de 3-16 atomes de C, qui peut être substitué le cas échéant par un hydroxy, carboxy, alcoxy $C_{1-6}$ ou alcoxycarboxyle $C_{1-6}$ ou benzylmercapto ou alkylmercapto $C_{1-6}$ ou benzylmercapto, un reste aryle de 6-10 atomes de C ou un reste aralkyle de 7-16 atomes de C, le système cyclique peut être substitué le cas échéant avec 1-3 atomes de fluor, chlore ou brome, un reste cycloalkyle de 3-8 atomes de C, un reste cycloalkylméthyle avec en tout 4-9 atomes de C,

$R^3$ est l'hydrogène, un reste acyle de 1-4 atomes de C, un reste alcényle ou alcynyle de 2-4 atomes de C et le cas échéant $R^1$ et $R^3$ ou $R^2$ et $R^3$ constituent ensemble un pont alkylène de 2-8 atomes de C dans lequel, le cas échéant une unité $CH_2$ est remplacée par $NR_1$, O ou S est OH, $OR_4$ ou

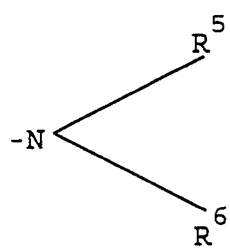

dans lesquels $R^4$ représente un reste alkyle de 1-4 atomes de C dans lequel les atomes de H peuvent être remplacés totalement ou en partie par des atomes d'halogène,

$R^4$ est un reste alkyle de 1-4 atomes de C dont les atomes de H sont remplacés le cas échéant par des atomes d'halogène, en partie ou totalement,

$R^5$ est l'hydrogène, un reste alkyle de 1-6 atomes de C, un reste hydroxy ou alcoxy de 1-6 atomes de C,

$R^6$ est l'hydrogène, un reste alkyle de 1-6 atomes de C, un reste phényle ou un reste cycloalkyle de 3-8 atomes de C ou le cas échéant $R^5$ et $R^6$, en incluant l'atome d'azote et le cas échéant un autre hétéroatome peuvent former un cycle de 4-8 maillons.

2. Composés selon la revendication 1, caractérisé en ce que,

$R^1$ représente un hydrogène ou un reste alkyle de 1-3 atomes de C,

$R^2$ est un hydrogène, un reste alkyle de 1-6 atomes de C, qui peut être non substitué ou substitué par un reste alcoxycarbonyle de 1-6 atomes de C, un reste phényle, aralkyle avec 7 ou 8 atomes de C ou cycloalkyle avec 3-7 atomes de C,

$R^3$ est un hydrogène ou un reste alkyle de 1-4 atomes de C ou $R^2$ et $R^3$ forment ensemble un pont alkylène de 2 atomes de C et

y est OH, $OR_4$ ou $NR_5R_6$ dans lesquels $R^4$ est un reste alkyle de 1-4 atomes de C, dont l'hydrogène peut être remplacé totalement ou en partie par du fluor et $R^5$ et $R^6$ indépendamment l'un de l'autre représentent l'hydrogène ou un alkyle de 1-4 atomes de C ou dans lesquels $R^5$ et $R^6$ forment avec l'atome N un cycle à 5-6 maillons, qui contient ou non un autre N ou O.

3. Composés selon les revendications 1 et 2, caractérisés en ce que Hal représente le brome, n le nombre 2 et $R^1$ l'hydrogène.

4. Composés selon les revendications 1-3, caractérisés en ce que

R2 représente l'hydrogène ou un reste alkyle de 1-4 atomes de C, qui peut être non substitué ou substitué par un reste alcoxycarbonyle de 1-2 atomes de carbone,

$R^3$ est un hydrogène, méthyle ou éthyle et

Y est OH, $OR^4$ ou $NR_5R_6$, dans lesquels $R^4$ est un reste alkyle de 1-4 atomes de carbone et

$R^5$ et $R^6$ indépendamment l'un de l'autre représentent l'hydrogène ou un reste alkyle de 1-4 atomes de C.

5. Composés selon les revendications 1-4, caractérisés en ce que $Hal_n$ représentent 2 atomes de brome en position 2' et 3' du cycle thiophène.

6. Préparation de composés selon les revendications 1-5, caractérisée en ce qu'on fait réagir un halogénure d'acide de formule générale II.

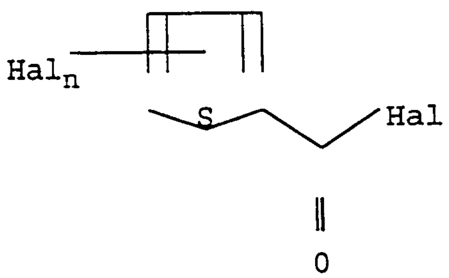

(II)

dans laquelle Hal = Chlore ou Brome et,
n est le nombre 2 ou 3
avec un composant amino de formule générale III.

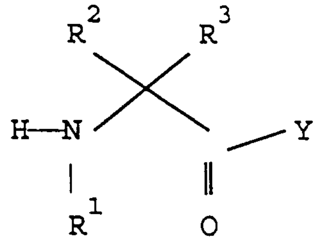

III

dans laquelle $R^1$, $R^2$, $R^3$ et Y ont les significations indiquées, en présence d'une base.

7. Moyen de lutte contre des germes pathogènes de plantes et de prévention de l'apparition de maladie des plantes, caractérisé en ce qu'il contient comme composants actifs au moins un composé selon la

revendication 1 en présence d'un véhicule approprié.

**8.** Procédé de préparation de moyens de lutte contre des germes pathogènes de plantes et de prévention de l'apparition de maladie des plantes, caractérisé en ce qu'il contient à côté d'un véhicule approprié comme composant actif au moins un composé selon la revendication 1 en présence d'un matériau véhicule approprié.

**9.** Utilisation de composés de formule I selon la revendication 1 pour lutter contre des germes pathogènes de plantes et pour prévenir l'apparition de maladie des plantes.

**10.** Procédé de prévention de l'apparition de maladie des plantes, caractérisé en ce qu'on traite les plantes, les parties de plantes, ou le lieu de leur croissance avec une quantité efficace d'un composé de formule I selon la revendication 1.

**Revendications pour l'Etat contractant suivant : ES**

**1.** Procédé de préparation de composés de formule générale I

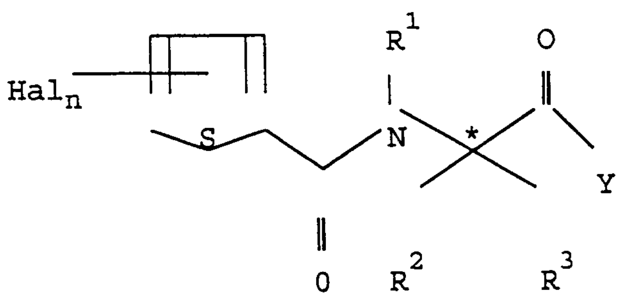

I

dans laquelle Hal représente le chlore ou le brome, n le nombre 2 ou 3,

* représente pour des restes $R^2$ et $R^3$ différents, soit un énantiomère, soit un racémate,

$R^1$ représente un hydrogène, un reste alkyle linéaire ou ramifié de 1-4 atomes de C,

$R^2$ est un hydrogène, un reste alkyle à chaîne linéaire de 1-16 atomes de C ou un reste alkyle à chaîne ramifiée de 3-16 atomes de C, qui peut être substitué le cas échéant par un hydroxy, carboxy, alcoxy $C_{1-6}$ ou alcoxycarboxyle $C_{1-6}$ ou benzylmercapto ou alkylmercapto $C_{1-6}$ ou benzylmercapto, un reste aryle de 6-10 atomes de C ou un reste aralkyle de 7-16 atomes de C, le système cyclique peut être substitué le cas échéant avec 1-3 atomes de fluor, chlore ou brome, un reste cycloalkyle de 3-8 atomes de C, un reste cycloalkylméthyle avec en tout 4-9 atomes de C,

$R^3$ est l'hydrogène, un reste acyle de 1-4 atomes de C, un reste alcényle ou alcynyle de 2-4 atomes de C et le cas échéant $R^1$ et $R^3$ ou $R^2$ et $R^3$ constituent ensemble un pont alkylène de 2-8 atomes de C dans lequel, le cas échéant une unité $CH_2$ est remplacée par $NR_1$, O ou S est OH, $OR_4$ ou

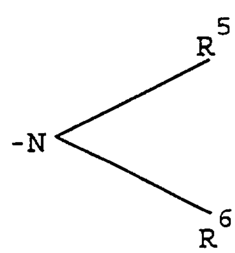

dans lesquels $R^4$ représente un reste alkyle de 1-4 atomes de C dans lequel les atomes de H peuvent être remplacés totalement ou en partie par des atomes d'halogène,

$R^4$ est un reste alkyle de 1-4 atomes de C dont les atomes de H sont remplacés le cas échéant

par des atomes d'halogène, en partie ou totalement,

$R^5$ est l'hydrogène, un reste alkyle de 1-6 atomes de C, un reste hydroxy ou alcoxy de 1-6 atomes de C,

$R^6$ est l'hydrogène, un reste alkyle de 1-6 atomes de C, un reste phényle ou un reste cycloalkyle de 3-8 atomes de C ou le cas échéant $R^5$ et $R^6$, en incluant l'atome d'azote et le cas échéant un autre hétéroatome peuvent former un cycle de 4-8 maillons

, caractérisée en ce qu'on fait réagir un halogénure d'acide de formule générale II.

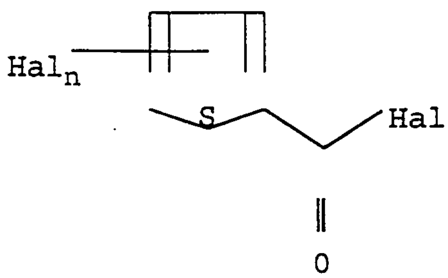

(II)

dans laquelle Hal = Chlore ou Brome et,
n est le nombre 2 ou 3
avec un composant amino de formule générale III.

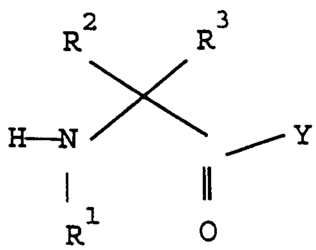

III

dans laquelle $R^1$, $R^2$, $R^3$ et Y ont les significations indiquées, en présence d'une base.

**2.** Procédé de préparation selon la revendication 1, caractérisé en ce que,

$R^1$ représente un hydrogène ou un reste alkyle de 1-3 atomes de C,

$R^2$ est un hydrogène, un reste alkyle de 1-6 atomes de C, qui peut être non substitué ou substitué par un reste alcoxycarbonyle de 1-6 atomes de C, un reste phényle, aralkyle avec 7 ou 8 atomes de C ou cycloalkyle avec 3-7 atomes de C,

$R^3$ est un hydrogène ou un reste alkyle de 1-4 atomes de C ou

$R^2$ et $R^3$ forment ensemble un pont alkylène de 2 atomes de C et

y est OH, $OR_4$ ou $NR_5R_6$ dans lesquels $R^4$ est un reste alkyle de 1-4 atomes de C, dont l'hydrogène peut être remplacé totalement ou en partie par du fluor et $R^5$ et $R^6$ indépendamment l'un de l'autre représentent l'hydrogène ou un alkyle de 1-4 atomes de C ou dans lesquels $R^5$ et $R^6$ forment avec l'atome N un cycle à 5-6 maillons, qui contient ou non un autre N ou O.

**3.** Procédé de préparation selon la revendications 1 et 2, caractérisés en ce que Hal représente le brome, n le nombre 2 et $R^1$ l'hydrogène.

**4.** Procédé de préparation selon les revendications 1-3, caractérisés en ce que

R2 représente l'hydrogène ou un reste alkyle de 1-4 atomes de C, qui peut être non substitué ou substitué par un reste alcoxycarbonyle de 1-2 atomes de carbone,

$R^3$ est un hydrogène, méthyle ou éthyle et

Y est OH, $OR^4$ ou $NR_5R_6$, dans lesquels $R^4$ est un reste alkyle de 1-4 atomes de carbone et

$R^5$ et $R^6$ indépendamment l'un de l'autre représentent l'hydrogène ou un reste alkyle de 1-4

atomes de C.

**5.** Procédé de préparation selon les revendications 1-4, caractérisés en ce que $Hal_n$ représentent 2 atomes de brome en position 2' et 3' du cycle thiophène.

**6.** Procédé de préparation de moyens de lutte contre des germes pathogènes de plantes et de prévention de l'apparition de maladie des plantes, caractérisé en ce qu'il contient à côté d'un véhicule approprié comme composant actif au moins un composé selon la revendication 1 en présence d'un matériau véhicule approprié.

**7.** Utilisation de composés de formule I selon la revendication 1 pour lutter contre des germes pathogènes de plantes et pour prévenir l'apparition de maladie des plantes.

**8.** Procédé de prévention de l'apparition de maladie des plantes, caractérisé en ce qu'on traite les plantes, les parties de plantes, ou le lieu de leur croissance avec une quantité efficace d'un composé de formule I selon la revendication 1.